# EUROPEAN PATENT SPECIFICATION

(11) **EP 1 686 378 B1**
(45) Date of publication and mention of the grant of the patent: **17.11.2010**
(21) Application number: 06001758.9
(22) Date of filing: 27.01.2006
(51) Int. Cl.: G01N 33/558, G01N 33/543

(54) **Fluid flow diffuser for immunological testing devices and method**
Durchflussdiffusor für immunologische Testvorrichtungen und Verfahren
Diffuseur de flux de fluide pour dispositifs et procédé de test immunologique

(30) Priority: 28.01.2005 EP 05001804; 25.03.2005 US 90463; 22.06.2005 US 158547; 26.10.2005 US 258636
(43) Date of publication of application: 02.08.2006
(73) Proprietor: DNT Scientific Research, LLC, San Diego, CA 92129 (US)
(72) Inventor: Naishu Wang, Poway, CA 92064 (US); David F. Zhou, Poway, CA 92064 (US)
(74) Representative: Fox-Male, Nicholas Vincent Humbert

(56) References cited:
- EP-A- 0 503 459
- EP-A- 0 903 584
- WO-A-01/27612
- WO-A-99/46591
- US-A1- 2001 055 542
- US-A1- 2003 096 424
- US-B1- 6 737 277

## Description

### Field of the Invention

This invention relates to apparatuses for analyzing body fluids and other fluids using immunochromatography, and more particularly to fluid test apparatuses for detecting antibodies or antigens in a near-patient setting, such as at the point-of-care especially, and/or in a clinical laboratory setting conducting large-scale, rapid, confirmatory examination.

### Background

Over past decades, the prior art has offered several types of rapid diagnostic testing techniques primarily for body fluids such as whole blood, serum, plasma, urine, spinal fluid, amniotic fluid, mucous, saliva, and the like for the presence of infection or other conditions such as pregnancy, abused drugs and cardiovascular disorders such as acute myocardial infarction (AMI).

The first of these tests were the Latex Particle Agglutination tests, then the Flow-Through tests leading to the current Lateral Flow Single Step test. Such tests typically utilize well known sandwich-type immunoassay techniques. In such tests a fluid sample or specimen is supplied from a subject which could carry at least one analyte, such as an antibody, or group of analytes which is specific to the condition being tested. The fluid specimen is exposed to a conjugate having at least one mobilizable binding member, such as an antigen (or antibody in some cases), which has immune-determinant(s) (or specific binding sites for the immuno-determinant(s) in certain cases) of the analyte in question. The binding member is conjugated to a visibly detectable label such as colloidal gold. During exposure of the specimen to the conjugate, an immuno-chemical reaction occurs wherein the analyte in question binds to the binding member to form the first affinity binding labeled analyte complex.

The fluid containing the labeled analyte complex is then directed to flow into a reaction membrane having a zone coated with at least one immobilized capture binding member which is similarly immuno-determinant of the analyte in question. As the fluid passes through the membrane, a second immuno-chemical reaction occurs wherein the labeled analyte complex binds to the capture binding member to form the second affinity binding immuno-sandwich complex. The accumulation of the secondly bound immuno-sandwich complex beyond a threshold amount in the zone generates a colored test line signal, the so-called "T-line". The reaction membrane typically has a second zone usually located further downstream from the T-line as an internal system control line, the so-called "C-line". The control line is used as an internal indicator of functional validity.

Unfortunately, depending on the type of condition being detected, these tests provide a typical accuracy of between 85% and 99%, falling short of the 99.9% or above accuracy generally considered to be necessary for a confirmatory test. The reasons for the insufficient accuracy are primarily due to the lack of overall higher sensitivity and specificity of the apparatus. Different samples may contain chemicals or particles which interfere with or inhibit the fluid flow or otherwise interfere with one or both of the affinity binding reactions. Prior apparatuses have attempted to enhance sensitivity or specificity by pretreating various parts of the apparatus with reaction or flow enhancing reagents, pH conditioning chemicals, or even non-specific adhesive blocking molecules which will "block-out" non-analyte molecules which might cause non-specific adhesion, or otherwise compete with the analyte in question for specific binding members, especially on the reaction membrane. These attempts have met with limited success in some types of testing, but do not provide the desired accuracy in many others. Also, pretreatment with two or more of the above pretreatments exacerbates the difficulties in obtaining uniform manufacturing due to potential incompatibilities between the pretreatment chemicals. For example, the pH conditioner might disrupt the effectiveness of the non-specific blocking member molecules. Or, the manufacturing step of pretreating with the second pretreatment chemical can dislodge some of the first pretreatment chemical.

Further, lot-to-lot variation in the manufacture of the test apparatuses can often lead to ambiguous results, such as false negatives as well as weak false positives, so-called "ghost lines" or "phantom lines". False negatives typically occur when non-specific molecules interfere with the first and/or second affinity binding actions. It has been found that non-analyte molecules can clump together in fluid samples that are not well mixed so that they temporarily prevent access between analytes and binding members. Even temporary interference can prevent an adequate number of labeled analyte complexes and/or ultimately immuno-sandwich complexes from forming. In this way, if a non-analyte molecule or clump of molecules blocks access between analytes and binding members for only a few seconds, it may be enough to induce a false negative result. Further, clumps of non-analyte molecules can carry an overabundance of the labeled mobilizable binding members to the second affinity binding site to generate a false positive.

Chemically non-uniform flows can result in flows having non-uniform first affinity binding by the time they reach the reaction membrane leading to inaccuracies. Such non-uniform flows can be caused by a number of factors. First, some portions of the fluid may flow faster than others from time to time. In those tests having deposits of dried reagent, faster flows tend to reach the dried reagent first. These flows along the initial fluid front tend to exhibit a greater degree of first affinity binding per unit fluid or at least uptake of mobilizable labeled binding members, and can potentially carry a greater concentration of clumps of non-analyte molecules which can carry away labeled mobilized binding members. Further, the deposit of dried reagent itself can exhibit portions of higher concentration than others resulting in similar chemical nonuniformity in the flow. Other flows having a lower than average concentration of analyte molecules, and/or having a greater concentration of non-clumped, non-analyte molecules which merely inhibit analyte binding but do not carry away mobilizable labeled binding members, exhibit less apparent first affinity binding. These flow and concentration disuniformities are responsible for many of the unsatisfactory results discussed above.

Therefore, although these prior devices provide a convenient, quick, economic, and simplified way to conduct such testing without requiring sophisticated instrumentation or trained professionals, in many settings these rapid tests are useful only for preliminary screening purposes, not as a confirmatory test. To this day, for example, the Western Blot Analytical Assay is the only one reliably used for the confirmatory detection of HIV infection in a clinical laboratory setting worldwide. Due to its multi-step manipulation and verification phases, completion of this type of assay takes days, if not weeks. Such a delay can unfortunately lead to further propagation of infectious pathogens such as HIV or other serious results, such as the metastasis of cancers. There is virtually no generally accepted practical or economical confirmatory rapid diagnostic testing technique for use in a point-of-care setting to rapidly detect serious diseases such as HIV infection and AMI, available in the market place today.

Therefore, there is a need to refine the accuracy and expedite the performance of prior immunoassay chromatographic rapid testing apparatuses to a higher and new level for use in the speedy and early detection and confirmation of the presence of pathogens or pathogenic conditions such as occurs with HIV infection, cancers and other disorders.

US Patent Specification 2003/096424 discloses sintered porous polymeric material.

### Summary

The present invention provides a flow immunoassay testing apparatus as defined in Claim 1.

The apparatus may include the features of any one or more of dependent Claims 2 to 16.

The present invention also provides a method as defined in Claim 17.

The method may include the features of any one or more of dependent Claims 18 to 21.

The instant embodiment provide an advanced, more efficient and more discriminative way of rapidly detecting the presence of cancer, infection or other conditions such as pregnancy, cardiovascular disorders, and abused drugs in body fluids through the use of fluid flow-based chromatographic immunoassay test devices and thus potentially avoid the long turn-around time required by use of separate assays using multiple tests such as in a Western Blot assay, or other sophisticated testing methods to achieve a confirmatory result.

The instant embodiments provide an improvement for flow-based immunological testing apparatuses for rapidly conducting a confirmatory immunoassay test for an analyte (or analytes) in question. These instant embodiments may also provide apparatuses that can be used, for example, in a point-of-care setting.

The instant embodiments provide an interrupting, porous, flow-diffusing structure interposed on a fluid path between a conjugate source and a reaction membrane. The diffusive structure causes numerous fluid furcations and convergences thereby improving the mixing of the fluid to provide a more uniformly dispersed fluid flow having a more uniform mixture of molecules, and thus a more uniformly high degree of first affinity binding before crossing into the reaction membrane and thereby enhancing the quality of the second affinity binding which results in an increase in the sensitivity and specificity (accuracy) of flow testing devices, and constitutes an advancement in performance over prior devices.

Some embodiments provide for pretreating the diffusive structure with a surfactant such as a non-ionic detergent, or other chemicals which can enhance fluid mixing.

In some embodiments there is provided a self-contained, multi-stage, programmed, interrupted downward flow, rapid confirmatory immunological test ("RCIT") apparatus contained in a single molded enclosure. The test can be performed in either a sandwich and/or competitive assay format. The apparatus can carry one or more chromatographic test strips in an inclined downward orientation forming "down-flow" test strips. The primary exposure of a fluid specimen to a specific antibody or antigen (or to a group thereof) conjugated to a label such as colloidal gold, or other type of label such as colloidal carbon, latex beads, or magnetic beads, etc. (hereinafter "conjugate") in a volume measured, reactive, buffered solution occurs in a first premix chamber or holding reservoir before flowing through the diffusive dam structure and on to contact the strips in a second chamber. The flow out of the incubation reservoir and into the strips is prompted by a combination of siphoning, gravity and capillarity action forces. In addition to mixing, the dam interrupts the flow to allow a short period of incubation resulting in an even greater degree of proper first affinity binding compared to prior rapid test technology.

In some embodiments, the down-flow strips are not of the type commonly used by prior lateral flow chromatographic testing methods in that they are not directly connected with any conjugate pad, but rather provide a reaction membrane which links with the outflow of the reservoir at the strip's top end. The down-flow strips can include a single layer of uniformly dispersed porous matrix material such as uniformly porous polyethylene commercially available from Porex Corporation of Fairburn, Georgia. Further, the down-flow strips can be adapted to carry fluid-diffusing pad to more thoroughly mix the fluid between the strip's top end and the test result signal generating zones. The strips can be held in an inclined rather than straight vertical position in order to reduce the height of the apparatus.

In some embodiments, a supply of aqueous mix buffer solution is held in a sealed tank until the sample specimen has been introduced into the apparatus. A user-manipulable member opens the tank allowing the mix buffer solution to be dispensed into a first chamber under atmospheric pressure. An absorbing pad at the bottom of a second chamber in contact with a lower part of at least one down-flow strip absorbs the excess buffer that has not been retained by the strip.

In still other embodiments, the first chamber contains a pad impregnated with an amount of lyophilized conjugate utilizing colloidal gold or other label.

In still other embodiments, after waiting for an adequate amount of time for the fluid mixture to flow through the strips, a second tank containing an amount of wash buffer solution is opened under the push of another manipulable member to wash down the remaining mixture through the first chamber and the strips and end the reaction at the signal zones on the strips. The absorbing pad absorbs the wash buffer and, along with the angled orientation of the strips, discourages reverse flow of fluid back up into the strips. Both of the manipulable members are movable, built-in components of the apparatus.

The premeasured buffer containing apparatus has many unique and advantageous features, including a self-contained measured volume mix and wash buffer tanks, a simplified program for successively opening these tanks during processing, interrupted flow from the first chamber to the second chamber, and diffusive action of the porous dam having numerous fluid furcations and convergences that provide more uniform mixing which improves accuracy and makes the apparatus well suited to rapid diagnostic point-of-care and/or near-patient testing.

In still other embodiments, there is provided one or more diffusive pad structures interposed between the conjugate pad and the reaction membrane of a lateral flow chromatographic test strip thereby improving its sensitivity and specificity (accuracy).

Some embodiments provide the in a flow immunoassay testing apparatus for testing a fluid specimen for the presence of an analyte, wherein said apparatus includes a source of labeled conjugate adapted to carry a binding member molecule bindable to said analyte, and a reaction membrane carrying a capture binding member molecule bindable to said analyte, an improvement which comprises: a first porous, diffusive structure located on a fluid flow path between a portion of said conjugate source and a portion of said reaction membrane, thereby creating a plurality of convergent fluid paths between said conjugate source and said membrane. In some embodiments said first diffusive structure has a first pretreatment condition. In some embodiments said first pretreatment condition is being pretreated with a surfactant. In some embodiments said surfactant comprises a non-ionic detergent. In some embodiments said first pretreatment condition is selected from the group consisting of: being pretreated with a surfactant; being pretreated with a pH conditioner, being pretreated with a non-specific adhesive blocking molecule, and having no pretreatment. In some embodiments said improvement further comprises a second diffusive structure, wherein said first diffusive structure has a first pretreatment condition and said second diffusive structure has a second pretreatment condition, and wherein said first pretreatment condition is different from said second pretreatment condition. In some embodiments said second pretreatment condition is selected from the group consisting of: being pretreated with a surfactant; being pretreated with a pH conditioner, being pretreated with a non-specific adhesive blocking molecule, and having no pretreatment. In some embodiments said first diffusive structure is made from material having a plurality of intersecting surface structures, wherein a junction between first and second of said structures is not substantially parallel. In some embodiments said first diffusive structure comprises a material selected from the group consisting of: fiberglass, cellulose and fibrous plastic and the like. In some embodiments there is no direct fluid flow contact between said conjugate source and said reaction membrane without passing through said first diffusive structure. In some embodiments said first diffusive structure comprises means for diverting an initial fluid front passing therethrough.

In some embodiments said apparatus further comprises: a first chamber shaped and dimensioned to accept said specimen and be subjectable to a supply of mix buffer solution and said source of labeled conjugate; a second chamber holding at least one test result signal generator responsive to an amount of said analyte bonded to said labeled conjugate; a first flow-interrupting dam comprising said first diffusive structure between said first and second chambers. In some embodiments said apparatus further comprises a second flow-interrupting dam spaced a distance apart from said first dam, said second dam comprising a second diffusive structure. In some embodiments said generator comprises a chromatographic test strip including a membrane oriented in an oblique, downward flow orientation, said membrane having at least one test zone. In some embodiments said strip is formed without a source of mobilizable labeled binding members. In some embodiments said membrane comprises a plurality of test zones adapted to provide a measurable basis for a qualitative result display. In some embodiments said membrane comprises a plurality of test zones adapted to provide a measurable basis for a quantitative result display. In some embodiments said second chamber comprises a plurality of chromatographic test strips adapted to provide a measurable basis for a quantitative result display. In some embodiments said generator comprises a plurality of test zones adapted to provide a measurable basis for a quantitative result display. In some embodiments said conjugate source comprises a lyophilized amount carried on a structure within said first chamber. In some embodiments said mix buffer solution is preformulated to carry an amount of said conjugate source in suspension. In some embodiments said strip further comprises a diffusive pad upstream from said at least one zone. In some embodiments the improvement further comprises means for triggering a dispensing of said supply into said first chamber. In some embodiments a volume of said supply of mix buffer solution is adjusted to create a minor outflow through said dam. In some embodiments the improvement further comprises an openable tank shaped and dimensioned to releasably hold said supply, wherein said tank in an open condition is in fluid communication with said first dam. In some embodiments said supply of mix buffer solution has a volume between about 200 microliters and about 300 microliters. In some embodiments the improvement further comprises a second supply of wash buffer solution having a volume between about 2.5 milliliters and about 3 milliliters.

In some embodiments said apparatus is a lateral flow chromatographic immunoassay strip, wherein said conjugate source is a conjugate pad and wherein said improvement further comprises: said diffusive structure being a diffusive pad located between a portion of said conjugate pad and said portion of said reaction membrane. In some embodiments said portion of said conjugate pad overlaps said first diffusive pad, and a portion of said first diffusive pad overlaps said portion of said reaction membrane.

Some embodiments provide that in an immunoassay flow testing apparatus wherein a fluid specimen in a buffered solution is first contacted with a colloidal conjugate in a first part of said apparatus, then applied to a chromatographic testing strip in a second part of said apparatus, an improvement which comprises a porous, flow interrupting and diffusing structure between said first and said second parts.

Some embodiments provide that in an immunoassay apparatus comprising a conjugate source adapted to carry a mobilizable binding member molecule specific to a particular analyte and a reaction membrane carrying a capture binding member molecule, an improvement which comprises: a first porous interrupting, diffusive structure located on a fluid flow path between a portion of said conjugate source and a portion of said reaction membrane, thereby creating a plurality of convergent fluid paths between said conjugate source and said membrane.

Some embodiments provide that in an immunoassay testing apparatus wherein a specimen is contacted with a reactive solution, the resulting mixture is applied to at least one chromatographic test strip, and said strip is contacted by a wash solution, an improvement which comprises: a first built-in manipulable member for releasing said reactive solution onto said specimen; and, a second, built-in manipulable member for releasing said wash solution onto said strip.

Some embodiments provide a method for premixing a measured buffer solution with a fluid specimen to form a mixture for dispensing to a chromatographic test strip, said method comprises: preloading said solution within an openable tank; placing a fluid specimen in a premix chamber; opening a passageway between said tank and said premix chamber, thereby dispensing said solution onto said specimen to create a mixture of said solution and said specimen; and, flowing said mixture from said chamber to said strip; and wherein said method further comprises delaying said flowing for an incubation period. In some embodiments the method further comprises washing said strip after passage of a time period after said flowing.

Some embodiments provide a method for conducting a fluid flow immunoassay, wherein said method comprises: diffusing a fluid mixture comprising a specimen, a mix buffer and a conjugate through a diffusive structure before said mixture reaches at least one reaction membrane.

Some embodiments provide a method for conducting a lateral flow immunoassay test which comprises: diffusing a fluid flow from a conjugate pad through a diffusive pad before said flow reaches a reaction membrane.

Some embodiments provide a method for conducting a fluid flow immunoassay which comprises: providing a fluid containing a specimen having an analyte; exposing said specimen to a labeled conjugate adapted to carry a binding member molecule bindable to said analyte; diffusing a flow of said fluid through a porous, fluid-diffusing structure before said flow reaches a reaction membrane carrying a capture binding member molecule bindable to said analyte.

Some embodiments provide a method for detecting a biological condition by means of a fluid flow nased chromatographic immunoassay rapid test on a sample of a patient's body fluid which comprises: exposing said sample to a strip having at least one line carrying an immunoassay complex component responsive to at least one inter-reactive component indicative of the presence of said condition in said patient; mixing said sample after said exposing, using a diffusive pad, to form a mixture; and, distributing said mixture to a reaction membrane which carries said at least one line. In some embodiments said mixing comprises evening a fluid front within said diffusive pad. In some embodiments the method further comprises pretreating said diffusive pad with a surfactant. In some embodiments said detecting occurs at an accuracy of at least 99.9%, thereby providing a confirmatory result.

### Brief Description of the Drawings

**Fig. 1** is a diagrammatical cross-sectional illustration of a first embodiment of a interrupted flow testing apparatus.
**Fig. 2** is a diagrammatical perspective view of the apparatus of Fig. 1.
**Fig. 3** is a diagrammatical cross-sectional side view of the apparatus of Fig. 1 showing the fluid flow through the dam.
**Fig. 4** is a diagrammatical microscopic illustration of fibers in a diffusive, flow interrupting pad material.
**Fig. 5** is a diagrammatical microscopic close-up illustration of a fiber junction of a diffusive, flow interrupting pad material of Fig. 4 taken at box 5-5.
**Fig. 6** is a diagrammatical microscopic illustration of the fiber junction of Fig. 5 where microscopic fluid flows are converging.
**Fig. 7** is a diagrammatical microscopic illustration of the fiber junction of Fig. 5 where microscopic fluid flows have converged and mixed.
**Fig. 8** is a diagrammatical cross-sectional side view of the apparatus of Fig. 1 showing the initial fluid flow into a dry dam.
**Fig. 9** is a diagrammatical cross-sectional side view of the apparatus of Fig. 1 showing the fluid flow out of a saturated dam.
**Fig. 10** is a perspective view of an alternate embodiment of the interrupted, diffusive downward flow testing apparatus having programmed dispensing of mix and wash buffers.
**Fig. 11** is a diagrammatical cross-sectional side view of the apparatus of Fig. 10.
**Fig. 12** is a diagrammatical perspective view of the tank fracturing pedestals of the apparatus of Fig. 10.
**Fig. 13** is a diagrammatical perspective view of a further alternate embodiment of the interrupted, diffusive downward flow testing apparatus having a spring loaded cover/support leg and more than one diffusive dam.
**Fig. 14** is a diagrammatical cross-sectional side view of the apparatus of Fig. 13.
**Fig. 15** is a perspective view of an alternate embodiment of the mix and wash buffer dispensing means having a puncturable bladder.
**Fig. 16** is a diagrammatical cross-sectional side view of the dispensing means for Fig. 15.
**Fig. 17** is a perspective view of another alternate embodiment of the mix and wash buffer dispensing means having crushable bladders having discharge tubes.
**Fig. 18** is a diagrammatical side view of the dispensing means for Fig. 17.
**Fig. 19** is a diagrammatical cross-sectional side view of yet another alternate embodiment of the mix and wash buffer dispensing means having a bladder crushable over an intimately engaging pedestal.
**Fig. 20** is a diagrammatical side view of the dispensing means for Fig. 19 in an open, crushed condition.
**Fig. 21** is a diagrammatical cross-sectional side view illustration of a test strip having a diffusive, flow interrupting pad. Fig. 21-28 are not part of the invention.
**Fig. 22** is a diagrammatical top view illustration of a test strip having a diffusive, flow interrupting pad and showing flow volume and conjugate concentration uniformization.
**Fig. 23** is a diagrammatical cross-sectional close-up side view illustration of test strip of Fig. 21 showing fluid flows into the dry portion of the diffusive pad.
**Fig. 24** is a diagrammatical cross-sectional close-up side view illustration of test strip of Fig. 21 showing downward fluid flows across the diffusive pad/reaction membrane border.
**Fig. 25** is a diagrammatical cross-sectional side view illustration of a test strip having two differently pretreated diffusive, flow interrupting pads straddling the conjugate pad.
**Fig. 26** is a diagrammatical cross-sectional side view illustration of a test strip having two differently pretreated diffusive, flow interrupting pads downstream of the conjugate pad.
**Fig. 27** is a diagrammatical side view illustration of a test strip using the diffusive pad and manufactured using a simple, partially overlapping pad structure.
**Fig. 28** is a diagrammatical exploded perspective view illustration of the arrangement order of different component pads of a test strip according to Fig. 27.

### Description of the Exemplary Embodiments

The instant apparatus is useful to rapidly and confirmatorily determine the presence of analyte in a sample or specimen. The sample can include, for example, body fluids such as whole blood, serum, plasma, urine, spinal fluid, amniotic fluid, mucous, saliva, and the like, or other fluids used in certain food and environmental testing.

Analyte, as used herein, refers to a compound or composition to be measured. The analyte can be any substance (antigen or ligand) for which there exists a naturally or genetically occurring specific binding member such as a binding molecule (e.g., an antibody or receptor).

Analyte also includes any antigenic substances, haptens, antibodies, and combinations thereof. The analyte can include a protein, a peptide, an amino acid, a ligand, a hormone, a steroid, a vitamin, a drug including those administered for therapeutic purposes as well as those administered for illicit purposes, a bacterium, a virus, and metabolites of or antibodies to any of the above substances. The analyte can also comprise an antigenic marker or antibody or receptor for single or multiple pathogenic conditions.

Representative analytes include steroids such as estrone, estradiol, cortisol, testosterone, progesterone, chenodeoxycholic acid, digoxin, cholic acid, digitoxin, deoxycholic acid, lithocholic acids and the ester and amide derivatives thereof; vitamins such as B-12, folic acid, thyroxine, triiodothyronine, histamine, serotonin, prostaglandins such as PGE, PGF, PGA; antiasthmatic drugs such as theophylline, antineoplastic drugs such as doxorubicin and methotrexate; antiarrhythmic drugs such as disopyramide, lidocaine, procainamide, propranolol, quinidine, N-acetylprocainamide; anticonvulsant drugs such as phenobarbital, phenytoin, primidone, valproic acid, carbamazepine and ethosuximide; antibiotics such as penicillins, cephalosporins, erythromycin, vancomycin, gentamicin, amikacin, chloramphenicol, streptomycin and tobramycin; antiarthritic drugs such as salicylate; antidepressant drugs including tricyclics such as nortriptyline, amitriptyline, imipramine and desipramine; as well as metabolites thereof. Additional therapeutic drugs include, for example, carbamazepine, free carbamazepine, cyclosporine, digoxin, FK778, lithium, N-acetylprocainamide, tacrolimus, free valproic acid, and the like, as well as the metabolites thereof.

Representative analytes also include drugs of abuse, and their metabolities, including amphetamines, methamphetamines, barbiturates, benzodiazepines (BZD), cannabinoids, cocaine (benzoylecgonine), opiates, phencyclidine (PCP), tricyclic antidepressants (TCA), methadone, propoxyphene (PPX), marijuana (THC), methylenedioxymethamphetamine (MDMA, or Ecstasy, or XTC), morphine, oxycodone, and bupromorphine. Representative drugs of abuse further include ethanol, heroin, hydromophone, oxymorphone, metapon, codeine, hydrocodone, dihydrocodiene, dihydrohydroxy codeinone, pholcodine, dextromethorphan, phenazocine and deonin.

Hepatic analytes include, for example, albumin bromocresol green (BCG) or purple (BCP), alkaline phosphatase, hepatitis B core antigen/antibody (anti-HBc), hepatitis B e antigen/antibody (anti-HBe), hepatitis B surface antigen/antibody (anti-HBsAg), hepatitis C virus (HCV), anti-HCV, direct bilirubin, gamma-glutamyl transpeptidase (GGT), antibody to Hepatitis A virus (HAVAb - IgG, HAVAb - IgM), hepatitis B surface antigen (HBsAg), lactate dehydrogenase (LD), neonatal bilirubin, prealbumin, total bilirubin, total protein, and the like.

Analytes related to pregnancy and fertility include, for example, human chorionic gonadotropin (hCG), beta-hCG, total beta-hCG, luteinizing hormone (LH), follicle stimulating hormone (FSH), dehydroepiandrosterone sulfate (DHEAS), estradiol, free estriol, total estriol, progesterone, prolactin, sex hormone binding globulin (SHBG), testosterone, and the like.

Analytes to determine blood disorders include, for example, B12, ferritin, folate, haptoglobin, and transferrin.

Analytes used to determine cardiac disorders include, for example, C-reactive protein (CRP), highly sensitive C-reactive protein (hsCRP), creatine kinase (CK), CK-MB, myoglobin, troponin, B-type natriuretic peptide (BNP), apolipoprotein A1, apolipoprotein B, and high density lipoprotein (HDL).

Cancer analytes include, for example, prostate specific antigen (PSA), free PSA, total PSA, fecal occult blood (FOB), acid phosphatase, alpha-fetoprotein (AFP), beta-2 microglobulin, CA 125™, CA 15-3™, CA 19-9™, carcinoembryonic antigen (CEA), PAP, pepsinogen, sqaumous cell carcinomas (SCC), and the like.

Analytes associated with inflammation and immunology include, for example, C3, C4, CRP, IgA, IgG, IgM, RF, and the like.

Analytes used to determine exposure to disease causative organisms include, for instance, rubella IgG, rubella IgM, toxoplasmosis IgG and IgM, cytomeglovirus (CMV) IgG and IgM, HTLV III, Anti-EBNA, mononucleosis, HAA, herpes, and anti-Streptolysin O.

Infectious disease analytes include microorganisms such as Streptococcus pyogenes, Staphylococcus aureous A, Endotoxin, Chlamydia, Syphillis, Gonococcus, Helicobactor pylori (H. pylori). Additionally, disease analytes include viral organisms such as hepatitis (HBV, HBsAg, HCV, HAA), hepatitis A virus, hepatitis B virus, hepatitis Non A-Non B, IgE, cytomeglovirus (CMV), herpes viruses, rubella viruses and the like. Further included, are, for example, toxoplasmosis, anti HTLV-I/HTLV-II, BSE, chagas antibody, CMV AB, CMV IgG, CMV IgM, CSF glucose, CSF protein, HIV AB, HIV-1/HIV-2 (rDNA), rotazyme II, and the like.

Analytes pertaining to endocrinology include, for example, thyroglobulin autoantibodies (anti-Tg), thyroid peroxidase autoantibodies (anti-TPO), C-Peptide, cortisol, HbA1c c (hemoglobin fraction), PTH, triiodothyronine (T3), free T3, total T3, thyroid hormone, thyroxine (T4), free T4, total T4, thyroid stimulating hormone (TSH), and the like.

Pancreatic analytes include, for example, amylase, lipase, and the like.

Veterinary analytes include, for example, Heartworm Ag, E. canis Ab, Lyme Ab, Giardia, parvovirus, FIV, FeLV, and the like.

Analytes can also include, for example, insulin, gamma globins, allergens, cystic fibrosis antigens, toxins, such as those associated with tetanus and animal venoms, and insecticides.

The precise nature of a number of analytes together with a number of examples thereof are disclosed in Litman, et al., U.S. Patent No. 4,299,916, issued November 10, 1981; and Tom, et al., U.S. Patent No. 4,366,241, issued December 28, 1982.

The signal provided to the user of the apparatus is provided by accumulation of a binding member such as a specific antibody and/or antigen; ligand and/or receptor ("binding molecule") conjugated to a label. In the instant embodiments, labels that produce a readily detectable signal are used. Thus, the instant embodiments provide colored labels which permit visible detection of the assay results without the addition of further substances and/or without the aid of instrumentation.

Examples of labels that can readily detected include, for example, dye sols, metal sols, nonmetal sols, colored latex particles, color indicators, colored matter encapsulated in liposomes, and the like.

Metal sols are disclosed in Leuvering, U.S. Patent No. 4,313,734, issued February 2, 1982 and Moeremans, et al., U.S. Patent No. 4,775,636, issued October 4, 1988, and comprise a metal, a metal compound, such as metal oxides, metal hydroxides and metal salts, or polymer nuclei coated with a metal or metal compound. The metal sols can comprise, for example, metals such as platinum, gold, silver and copper. Alternatively, or additionally, the metal sols can comprise metal compounds, such as, for example, silver iodide, silver bromide, copper hydrous oxide, iron oxide, iron hydroxide or hydrous oxide, aluminum hydroxide or hydrous oxide, chromium hydroxide or hydrous oxide, vanadium oxide, arsenic sulphide, manganese hydroxide, lead sulphide, mercury sulphide, barium sulphate and titanium dioxide.

Nonmetal sols, such as carbon sols and their use are described in Kang, et al., U.S. Patent No. 5,559,041, issued September 24, 1996. Nonmetal colloidal particles, such as selenium particles, are disclosed in Yost, et al., U.S. Patent No. 4,954,452, issued September 4, 1990. Other nonmetals that can be used include elements within Group VIB, of the Periodic Table, such as sulfur, and tellurium.

Labels can also be formed from dye polymers, whereby dye molecules, or chromogenic monomers, are polymerized to form a colored polymer particle. Examples of such dyes include Congo red, Trypan blue, and Lissamine blue.

Organic polymer latex particles are disclosed in Tarcha, et al., U.S. Patent No. 5,252,459, issued October 12, 1993. Such particles can comprise a plurality of non-chromophoric monomers.

Particulate labels comprising a dye or other colored substance enclosed in liposome sacs are described in Campbell, et al., U.S. Patent No. 4,703,017, issued October 27, 1987; and Rosenstein, U.S. Patent No. 5,591,645, issued January 7, 1997.

The apparatuses described in these embodiments use test strips, dams and/or pads that can comprise a dry porous material. By "porous" it is meant that the matrix is composed of a material into which fluids can flow and can pass through. Representative materials include nylon, plastic, fiber containing paper, such as filter paper, chromatographic paper, and the like, nitrocellulose, glass fibers, polysullfone, polyvinylidene difluoride, polyurethane, and other porous polymers, polysarcharides, (e.g., cellulose materials, such as paper and cellulose acetate), silica, inorganic materials, such as deactivated alumina, diatomaceous earth, MgSO₄, or other inorganic finely divided material conveniently substantially uniformly dispersed in a porous polymer matrix, with polymers such as vinyl chloride, vinyl chloride-propylene copolymer, and vinyl chloride-vinyl acetate copolymer; cloth, both naturally occurring e.g., cotton and synthetic (e.g., nylon cloth), porous gels, (e.g., silica gel, agarose, dextran, and gelatin), polymeric films, (e.g., polyacrylamide), and the like. In exemplary embodiments, the test strips comprise POREX CHEMISTRY A and/or POREX CHEMISTRY K membranes commercially available from Porex Corporation, Fairburn, GA, and/or NOVYLON brand membrane commercially available from Cuno Incorporated, Meriden, CT.

The exemplary embodiments will be described in connection with the detection of HIV in a fluid specimen. Those skilled in the art will readily appreciate adaptation of these embodiments to detect other analytes indicative of other pathogens, or pathogenic conditions within body, food or environmental fluid samples.

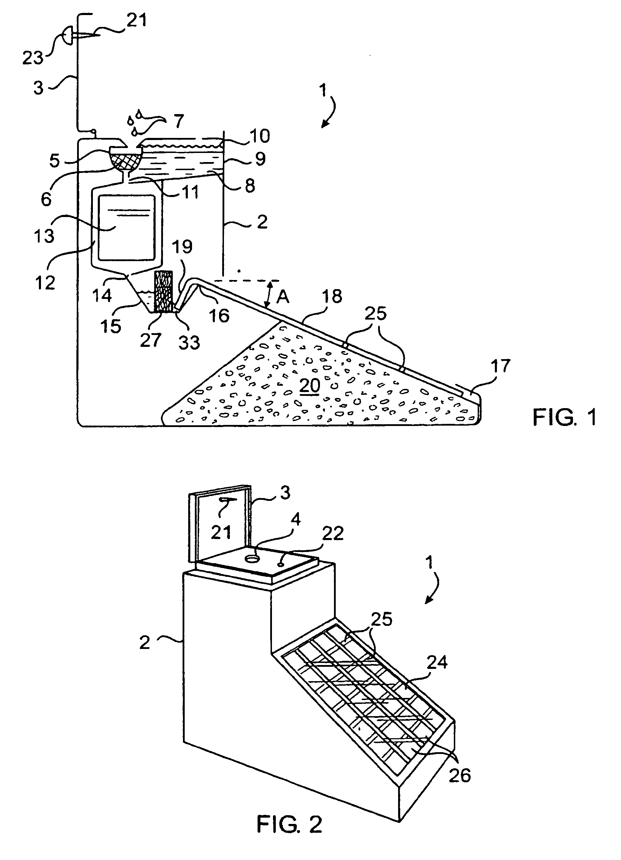

Referring now to the drawing, there is shown in Figs 1-2 a first example embodiment of an immunoassay testing apparatus 1 packaged in a molded plastic enclosure 2 topped by a sealing cap 3. In the upper region of the apparatus, and immediately under a ceiling bole 4 is a sampling well 5. The internal wall of the well is funnel-shaped, and retains some filtration material 6. The geometry of that wall, whether in the form of a V or a U, has a portion of a relatively low pitch so that when a drop, typically about 40 to 100 microliters, of fluid specimen 7 such as whole blood, or saliva runs along the wall, particles and adhesive matters are separated from the fluid component of the specimen. A supply of aqueous mix buffering solution 8 is held in a tank **9** along side the sampling well. In some embodiments, the mix buffer solution comprises Borax: 2-3%; nonfat dry milk: 0.2-0.5%; Sucrose: 0.05-0.1%; NaN3: 0.4%; Rabbit IgG: 2%; Goat IgG: 2%; Human IgG: 0.2%; Tween 20: 0.5% and the like.

The tank **9** has a top opening hermetically sealed by a membrane **10,** and a dispensing port **11** in a lower region leading to a first chamber **12** in a first analytical part of the apparatus. The first chamber is located immediately below the sampling well and receives the fluid component and is subjectable to the buffering solution. The dispensing of the buffer solution **8** out of the tank **9** is triggered by puncturing the membrane **10.** The puncturing is accomplished by a prong **21** which extends from the underside of the cap **3** and passes through an aperture **22** in the roof of the enclosure. The prong is normally held into a retracted position during storage and shipment of the apparatus, but can be moved to an extended position by manipulating a knob **23** on the outside of the cap. The prong is positioned, shaped and dimensioned to extend sufficiently through the aperture **22** into rupturing contact with the membrane **10.** The buffer solution **8** combines with the fluid components of the specimen that come down from the sampling well to form a mixture which flows into the first chamber **12.**

The first chamber **12** holds a conjugate pad **13** made of porous inorganic material such as fibreglass or 3M paper, and the like which has been coated with a soluble, lyophized amount of conjugate which includes at least one mobilizable binding member, specific to the analyte or analytes in question, conjugated to a visibly detectable label such as colloidal gold. In HIV testing, for example, the mobilizable binding member can include immuno-determinant epitopes of the HIV virus such as p18, p24, p32, gp36, gp41, p51, p55, p65, gp120, gp160 and subtype o. In this way the pad provides a source of labeled conjugate specific to the analyte or analytes in question. While the fluid mixture is exposed to the conjugate, an immuno-chemical reaction occurs wherein analytes in question from the specimen begin binding to the mobilizable binding members in the conjugate to form the first affinity binding labeled analyte complexes. It should be noted that alternately, the mix buffer solution can carry the an amount of conjugate in suspension. However, this can decrease the shelf life of the apparatus.

An outlet **14** at the bottom of the first chamber **12** leads to a pit **15** which forms a flow interrupting, incubation reservoir where the fluid **30** accumulates behind a porous dam structure **27** made of diffusive material whose function will be described in greater detail below. In general, the dam further mixes the fluid passing through it so that the fluid has greater chemical uniformity and is provided a temporary, but longer incubation time for the first affinity binding to reach a maximum.

Having passed through the porous, diffusive dam **27,** the fluid accumulates in the downstream portion **33** of the pit until it flows over an escape port **16** leading to a second chamber **17** in a second analytical part of the apparatus which holds at least one chromatographic testing strip **18** oriented in an oblique, downward flow orientation, and which can be referred to as a "down-flow" strip. An upper edge **19** of the strip **18** extends into the downstream region **33** of the pit allowing the fluid to flow into and through the strip under the combined forces of gravity, siphoning and capillarity. The flow is enhanced by an absorbing pad **20** positioned in the bottom of the enclosure and in contact with a lower portion of the strip **18.** The size of the absorbing pad is selected to accommodate the combined volume of the fluids within the apparatus. It should be understood that the diffusive, interrupting dam **27** is located between the conjugate source and the reaction membrane. In this way, there is no uninterrupted fluid flow contact between the conjugate source and the reaction membrane, but rather the fluid must pass through the diffusive dam before reaching any reaction membrane.

Each down-flow strip **18** can be positioned in an inclined position at a pitch angle **A** of at least 15 degrees from the horizontal. The down-flow strip differs from those common chromatographic strips typically used in lateral flow tests. The primary difference is that each down-flow strip does not provide a conjugate pad and is without a source of mobilizable labeled binding members, but does provide a reaction membrane having a number of test lines or zones **25** each coated with at least one immobilized capture binding member having an affinity for binding to the analyte or analytes in question. Immobilization techniques include reel to reel dispenser of binding members such as binding molecules. The down-flow test strips are then blocked to facilitate the test. Such a blocking procedure includes, for example, treating the strips with a buffer comprising Triton X-100: 1%; Polyvinyl Alcohol (PVA) (30,000 - 70,000 mw) or Polyvinyl Pyrrolidone (PVP) (10,000 mw): 1 - 1.5%; and Sugar: 0.2% and the like. It is important to note that by providing the conjugate and the reaction membrane in its constant, dried form, the apparatus can have a shelf life in excess of 2 years.

As the fluid passes through the membrane, a second immuno-chemical reaction occurs wherein the labeled analyte complex binds to the capture binding member to form the second affinity binding immuno-sandwich complex. The accumulation of the secondly bound immuno-sandwich complex beyond a threshold amount in the zone creates a colored test line. In this way the zones act as a test result signal generator. A transparent window **24** sealed to the enclosure provides a direct viewing of the test line zones **25** on a number of down-flow strips **26.**

The reaction membrane region of the down-flow strip can be made from a single layer of substantially uniformly dispersed porous matrix material such as porous polyethylene commercially available from Porex Corporation of Fairburn, Georgia. The substantially uniform dispersion of pores in the material reduces the negative effect of lot-to-lot variation present in prior membranes. This greater predictability in how a given strip will perform further enhances the sensitivity and accuracy of the test.

It should be noted that the buffer solution and fluid components of the specimen provide the volume of fluid necessary to fill the incubation reservoir and thus, regulate the transfer of the specimen through the apparatus. By adjusting the volume of buffer solution to what is necessary to create a minor and/or relatively slow overflow of the reservoir, excessive flooding and rapid flow through the strip is avoided. Further, it should be noted that the escape port **16** acts as a means for restricting the flow therethrough. It is further understood that the location, shape and capacity parameters of the pit **15** and the shape and dimension parameters of the dam **27** can be adjusted to provide a temporary, but longer incubation time, or better mixing of the mixture depending on its anticipated volume and viscosity.

Within about 0.5-2 minutes, the relatively slow speed of the flow of the mixture through the dam **27** and over the escape port **16** caused by the combination of siphoning, gravity and capillarity action forces, promotes a maximum degree of the second affinity binding on the down-flow strips. The two maximized affinity binding steps in turn maximize the diagnostic sensitivity of the RCIT apparatus technology.

Further, such sensitivity allows for the creation of a quantitative assay strip having a number of lines where each line is selected to appear at a different concentration threshold. In this way, the apparatus carrying one or more quantitative assay strips can provide the digital display of a quantitative result. Further, the apparatus can be adapted to include an electronic sensor for automatically detecting the result and an electronic display, such as a liquid crystal display (LCD) for displaying the results. Each of a plurality of strips can be coated with a number of corresponding antigenic epitopes (or polymers, proteins, polypeptides, etc.) which are immuno-determinant analytes for the pathogen in question, such as the HIV or the condition in question such as cancer. In HIV confirmatory detection, the appearance of at least two epitope lines on a down-flow strip will not only confirm the presence of HIV, but will also give a qualitative, analytical indication of the type of antibodies present in the specimen which can change during different periods of HIV infection and/or Acquired Immune Deficiency Syndrom (AIDS). Additionally, each strip can include a control line, working as an internal system control indicator. Therefore, any positive result of HIV detection shown by this RCIT method can include at least three (3) lines appearing in the test reaction window.

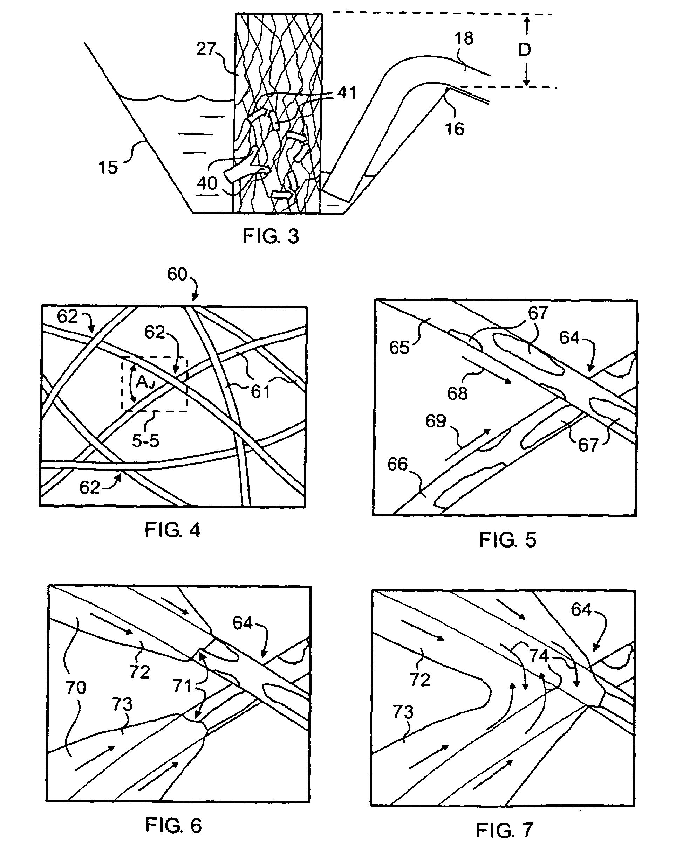

Referring now to Figs. 3-7, the detailed operation of the interrupting, diffusive dam structure will now be described. The shape and dimensions of the pit **15,** and the size and location of the dam **27** are further selected to avoid fluid flow over the top **31** of the dam. An example is selecting the elevation of the top of the dam to be a distance **D** above the elevation of the escape port **16.**

The diffusive nature of the dam **27** causes the flow to separate at the fluid front into a plurality of branches or furcations **40** which, when these furcations converge and join together again, do so from different directions. The convergence from different directions **41** causes a mixing across the fluid front and hence the entire specimen as it flows through. This mixing can cause the break-up of clumps of non-analyte molecules which may carry mobilizable labeled binding members, and/or clumps of the analyte itself and/or clumps of labeled analyte complexes which could carry additional mobilizable labeled binding members. The breaking up of these clumps help the to reduce the possibility of a false positive result. The mixing also reduces the differences in the concentrations of non-analyte molecules and labeled analyte complexes so that they are spread more evenly. Once the fluid passes through the dam, the concentrations have superior uniformity which leads directly to giving the labeled analyte complexes a greater opportunity to form the second affinity binding and thereby helping to reduce the possibility of a false negative result.

In addition, the diffusive action of the porous dam **27** automatically further delays or interrupts the flow from the first chamber **12** to the reaction membrane of the down-flow strip **18** providing more incubation time for the first affinity binding to reach a maximum, thereby increasing the overall sensitivity and specificity of the test.

Referring now to Fig. 4, there is shown a portion **60** of a diffusive dam structure made from relatively non-reactive, porous material such as fiberglass, cellulose, polysullfone, NYLON brand material, polyethylene, NOVYLON brand material, POREX CHEMISTRY K brand material, POREX CHEMISTRY A brand material, FILTRONA brand material, and the like, all commercially available. The material is selected to have intersecting surface structures such as fibers **61** oriented substantially differently to one another to create a plurality of junctions **62** where there is typically an angle **A**_{J} formed between the two intersecting structures at their junction where the angle is not 0 degrees. In other words, at the junction, the fibers should not be substantially parallel so that they provide intersecting surfaces which, through surface tension forces cause the fluid to branch into furcations and converge causing a more thorough intermixing. It is important to note that the viscosity of the fluid can be a factor in determining the optimum density of fibers and hence the number of junctions required in a given volume of material. It has been found that fiberglass material commercially available from JBC of Elyria, Ohio provides an adequate number of junctions for many applications. Other non-fibrous porous materials can also be used that provide intersecting surface structures to cause fluid furcation and intermixing, and provide the capability for carrying a dried surfactant.

The portion of the diffusive dam structure can be pretreated with a surfactant by immersing the dam material during manufacturing into an amount of liquid surfactant so that it penetrates substantially all of the pores of the dam material. The dam material is then dried. This leaves a residue of the surfactant on the intersecting surface structures. As shown in Fig. 5, the junction **64** of two intersecting surface structures such as fibers **65,66** having dried surfactant residue **67** thereon creates two convergent fluid paths **68,69.** As shown in Fig. 6, fluid **70** flowing through the dam will at its fluid front **71** have an affinity for separating into branches or furcations **72,73** which each tend to flow along the fluid paths formed by each surfactant treated fiber. As shown in Fig. 7, the furcations **72,73** will meet at the fiber junction **64** and their respective velocities will cause an intermixing as indicated by flow lines **74.**

An exemplary surfactant is a detergent such as polyethylene glycol sorbitan monolaurate commercially available under the brand name TWEEN 20 from Sigma-Aldrich Corporation of St. Louis, Missouri. Other detergents are acceptable such as TRITON X-100 brand, TRITON X-114 brand, TWEEN 80 brand, and sodium dodecyl sulfate ("SDS") detergents also available from Sigma-Aldrich Corporation. Depending on the test being conducted, other anionic, cationic, non-ionic and Zwitterionic detergents may also be acceptable.

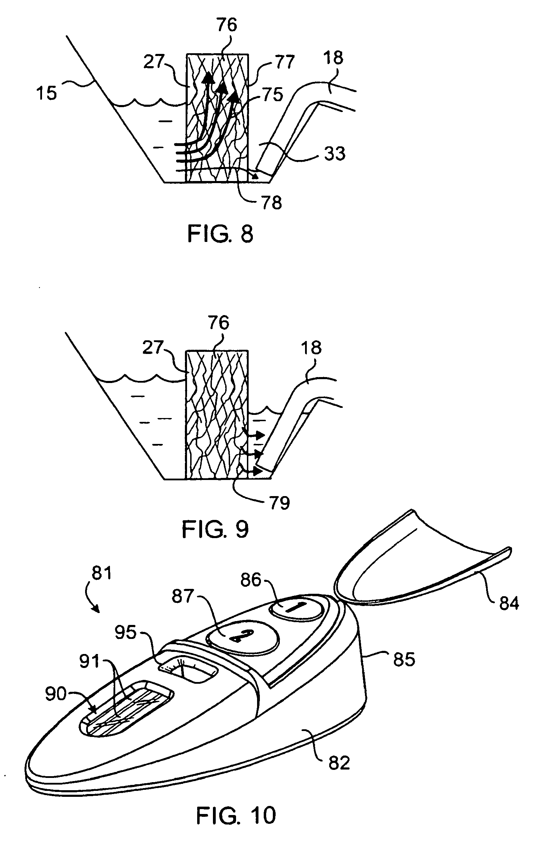

Referring now to Fig. 8, the fluid flow being driven primarily through capillary forces will tend toward fully saturating the diffusive dam **27** before any substantial flow exits the dam. This preferential fluid flow direction is primarily due to the differential surface tension caused by the surfactant treatment of the surface structures in the dam. Arrows **75** indicate that the predominant flow will be first toward the dry top portion **76** of the dam rather than out of the downstream side **77** of the dam and into the downstream region **33** of the pit **15** as indicted by the smaller flow arrow **78.** In this way the dam acts partially as a reservoir for delaying or interrupting the flow giving more time for first affinity binding to occur. Depending on the chemistry of the test being performed and the viscosity of the sample being tested, the flow can be delayed between about 1 and about 40 seconds. For relatively low viscosity samples such as urine, the flow can be delayed for between about 2 and about 3 seconds.

Referring now to Fig. 9, as the dam **27** eventually becomes saturated, the fluid pressure, due to continued capillary action and gravity, builds at the downstream side until the flow begins to exit **79.** This relatively rapid breakdown of the surface tension barrier at the downstream side causes further mixing and leads to a more evenly mixed exiting fluid front. Further, because of the vertical structure of the diffusive dam **27,** the initial fluid front generally stagnates at the top portion **76** of the dam while the fluid front exiting the dam is fluid from the more predictably mixed, non-initial-front fluids at the bottom of the dam. In this way, the surfactant treated dam provides an additional, initial fluid front bypass function or a means for diverting the initial fluid front toward the top portion of the dam rather than out the bottom potion as would be dictated by gravitational forces alone. This increases the uniformity of the first amount of fluid reaching the reaction membrane, which enhances the sensitivity and specificity of the affinity bindings described above.

Pretreatment of the dam can also be in the form of pH conditioning chemicals, and non-specific adhesive blocking molecules which will selectively filter or "block-out" unwanted non-analyte adhesion molecules, typically proteins, which can non-specifically interfere and/or compete with the analyte in question in either or both of the first and second affinity binding stages of the test.

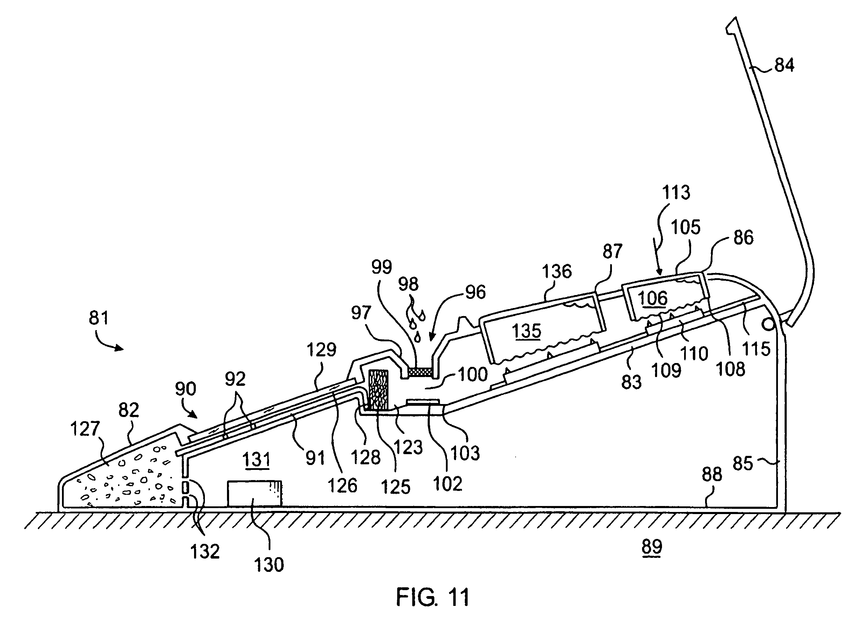

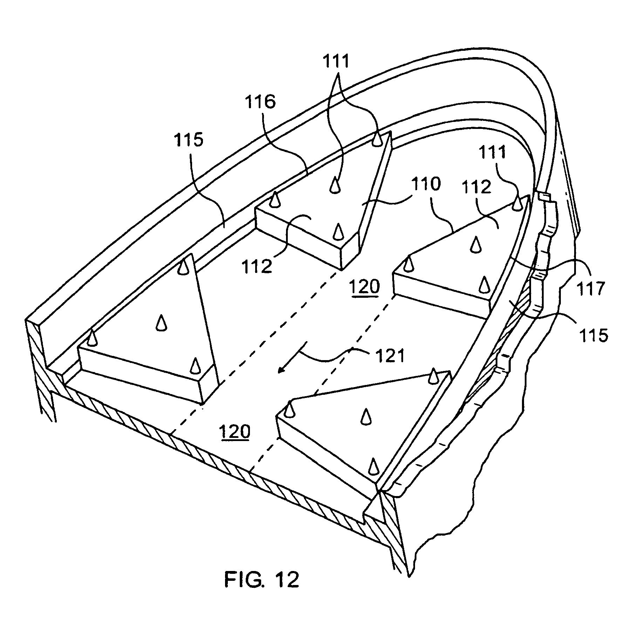

Referring now to Figs. 10-12, there is shown a further embodiment of an immunoassay testing apparatus **81.** The apparatus is packaged in a molded plastic enclosure **82** having an internal base pan **83.** A retractable protective cover **84** is hingedly mounted at the back end **85** of the enclosure. The cover is in a closed position during storage and shipment of the apparatus to protect a pair of operation buttons **86,87** from being inadvertently pressed, but can be flipped open for testing. The enclosure is shaped to have a generally planar bottom support surface **88** to support the apparatus upon a level surface **89** so that a test station **90** holding one or more down-flow testing strips **91,** having test line zones **92,** is oriented in an inclined position from the horizontal.

In the medial region of the apparatus, and immediately under a ceiling hole **95** is a sampling well **96** having a funnel-shaped internal wall **97** and a filter screen **99** to help separate particles and adhesive matters from the fluid component of a specimen **98** such as whole blood or saliva. The sampling well **96** leads downwardly to a first, premix chamber **100** which receives the fluid component of the specimen in a first analytical part of the apparatus. The specimen drops upon a conjugate pad **102** located on the floor **103** of the premix chamber thereby exposing the specimen to a source of conjugate. The viscosity of blood or saliva for example generally prevents it from flowing on its own through to the rest of the apparatus. The shape and dimensions of the first chamber can be adapted for other different viscosity fluid specimens.

The analytical testing reaction is initiated by opening a first tank **105** containing a measured amount of mix buffer solution **106.** The tank is formed by a generally inverted cup-like button structure **86** made from a durable, rigid, fluid impermeable material such as hard plastic. The cup structure rim **108** is breakably thermo-sealed by a foil-plastic membrane **109.** In its pretest position the mix buffer tank rests above a pair of puncturing pedestals **110** each having a plurality of prongs **111** extending upwardly from the pedestal upper surface **112.** The prongs are located near the periphery of each pedestal to help cause fracturing of a pliable foil-plastic membrane. The tank is opened by downward pushing manipulation **113** of the first button **86** which causes the tank to be lowered onto the pedestals, fracturing the foil-plastic membrane. Downward motion is stopped by a peripheral lip **115** straddling the outer lateral edges **116,117** of the pedestals. The pedestals are laterally spaced apart to form a fluid channel **120** therebetween allowing the solution to flow out of the tank and down **121** toward the premix chamber **100.**

The mix buffer solution **106** is dispensed into the premix chamber **100** to contact the conjugate pad **102** and mix with the specimen to form a fluid mixture and initiate the first affinity binding reactions. In this way the premix chamber is subjectable to the mix buffer solution which can be characterized as a reactive solution. The button and openable tank forms a built-in manipulable member for triggering the dispensing of the mix buffer solution onto the specimen. The amount or volume of mix buffer solution **106** is selected to adequately react with the amount of specimen. In this embodiment the volume of mix buffer is between about 200 and 300 microliters, and can be about 250 microliters.

Similarly to the previous embodiment, the mixture collects in a pit **123** formed into the floor **103** of the premix chamber **100.** The pit is shaped and dimensioned to have a given capacity to form a temporary incubation reservoir behind a porous, diffusive dam structure **125.** The mixture flows slowly and diffusively under the combined effect of gravity, capillarity, and siphoning forces through the dam and into one or more down-flow testing strips **91** held in a second chamber **126** in the test station **90** and eventual on to a flow absorbing pad **127** contacting the lower edge of the strips. The upper edge **128** of the strips extend into the pit **123** and contact the downstream side of the dam **125.** A transparent window **129** sealed to the enclosure provides for direct viewing of the strips **91.** A block of desiccant **130** is held in a third chamber **131** in the enclosure and is in communication with the absorbing pad through holes **132** to help extend shelf life. Additionally, another embodiment provides that the entire apparatus is kept in a sealed hermetic aluminum plastic foil pouch bag until use.

The apparatus also provides for a supply of a stop wash buffer solution **135** to the down-flow strips in order to stop the reaction in the strips and to carry away lingering chemicals and residue which could serve to obscure the lines formed in the zones **92** on the strips **91** and also to remove any other non-specific materials from the reaction area. For example, one such stop wash buffer comprises: Tween 20: 1 %; Glycerol: 0.5%; Glycine: 5- 20mM; and NaN3 : 0.02% and the like.

The stop wash buffer solution **135** is contained in a second tank **136** located upstream from the premix chamber **100** and which is openable in a manner similar to the mix buffer tank 105 thereby providing a built-in, manipulable member for releasing the wash solution onto the down-flow strips.

The wash buffer can be applied after a certain programmed waiting period which allows for the mixture to be drawn through the strips to an adequate degree. The waiting time can of course be dependent on the type of test being performed. For HIV detection the waiting period can be between about 0.5 and 2 minutes. The amount of wash buffer solution is selected to adequately wash the down-flow strips without unduly increasing the bulk of the apparatus, and in this embodiment is between about 2 and 3 milliliters, and can be about 2.5 milliliters. In this embodiment the volume of wash buffer is about ten times that of the mix buffer. The flow of the stop wash buffer occurs relatively more quickly than the first flow of the mixture. The wash buffer, having about ten times the volume of the mixture, rapidly penetrates the already moistened dam and flushes out the non-specific binding caused by non-specific materials in the reaction area. This action helps to maximize the specificity of the present apparatus to provide RCIT.

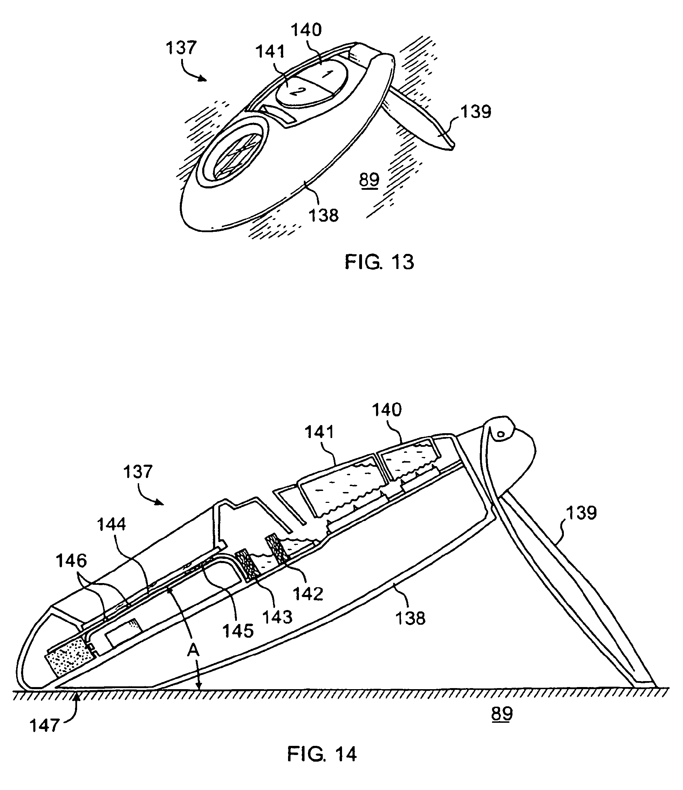

It is understood that both of the tanks **105,136** are in fluid flow communication with the channel **120** which is in fluid flow contact with the premix chamber **100** which is in fluid flow contact with the dam **125** which is in fluid flow contact with the strips **91,** meaning there is a fluid path from the tanks, through the premix chamber **100,** to the test station **90.** Referring now to Figs. 13 and 14, there is shown another alternate embodiment of an immunoassay testing apparatus **137** which operates similarly to the previous embodiment. The apparatus is packaged in a compact and rounded molded plastic enclosure **138** and includes a spring-loaded retractable protective cover **139** hingedly mounted at the back end of the enclosure. The cover is in a closed position during storage and shipment to protect a pair of operation buttons **140,141,** but is flipped open position for testing. In this embodiment the cover swings nearly 270 degrees around so that it forms a support leg **139** in the open position to orient the apparatus at a favorably inclined angle A upon a level surface **89.** The housing also has a flattened underside outer surface portion **147** at the opposite, front end of the enclosure to provide greater stability.

This embodiment is further characterized by two, diffusive porous dam structures **142, 143** spaced a distance apart from one another. In this way, the upstream diffusive dam **142** can have a different pretreatment condition than the downstream diffusive dam **143.** For example, the upstream dam can be pretreated to have a pH conditioner; and the downstream dam be pretreated to have a non-specific adhesive blocking molecule and a surfactant.

By splitting the pretreatment chemicals between two or more dams, the test apparatus designer is not only given increased accuracy, but also more control and predictability over the reactions occurring at various stages of the testing process. Also, the designer is given the flexibility in assembly to inexpensively use the same housing design for a number of different tests depending on the type of dams, their pretreatment, the type of strips, the type of conjugate pad and buffer solutions which can easily be swapped. Also, using two or more pretreated dams potentially avoids manufacturing inconsistencies such as the second pretreatment chemical dislodging some of the first pretreatment chemical on a single dam.

It should be noted that in the multi-diffusive dam embodiments, no pretreatment can be a pretreatment condition. For example, the upstream dam **142** may simply be a fiberglass filter without any surfactant, pH conditioner or non-specific adhesive blocking molecule. In this case its pretreatment condition would be "unpretreated". This pretreatment condition would be different from the pretreatment condition of the downstream dam **143** which could have a dried surfactant pretreatment condition.

This embodiment shows that the down-flow strips **144** can be further adapted to have a pad 145 made from diffusive material to further mix the fluid as it flows down the strip. The diffusive pad is located upstream from the signal generating zones **146** carrying the immobilized binding members. Further, the pad may be pretreated similarly to the dams described above.

Again it should be noted that the increased accuracy provides the ability to make multiline strips for detecting different analytes in a single specimen such as for detecting multiple major epitopes, i.e., antigenic determinants of an endogenious disorder such as AMI, or a single exogenous pathogen, or pathogenic organism such as bacteria, viruses such as HIV, parasites, rickettsia, and the like.

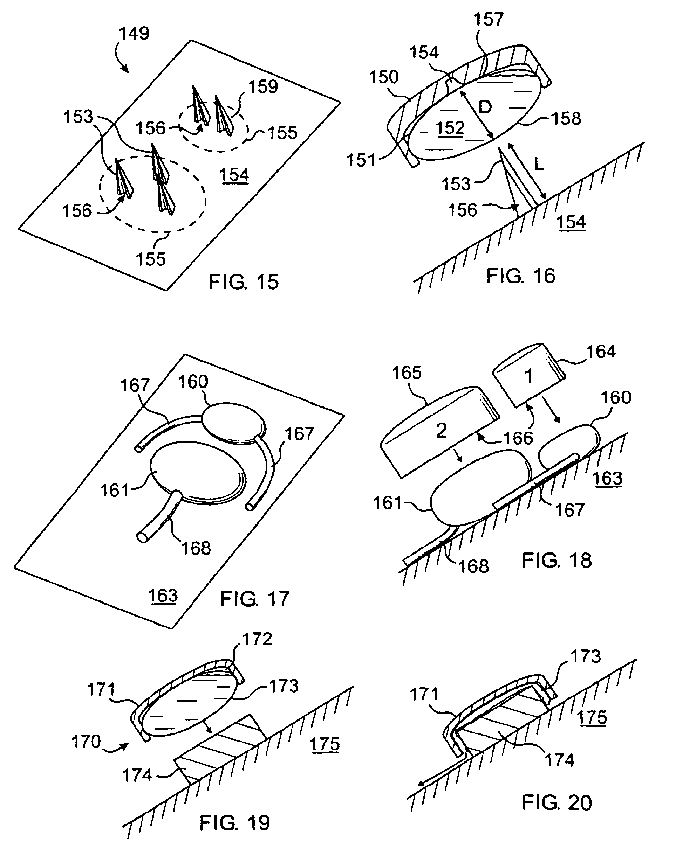

Referring now to Figs. 15-20, there are shown alternate embodiments of the openable mix and wash buffer tanks. These embodiments provide means for dispensing the volume of solution from the tanks in a manner which is substantially uniform across a manufacturing run of many test apparatuses, while keeping the apparatus inexpensive to manufacture, and making the tanks resistant to inadvertent opening.

As shown in Figs. 15-16, there is a first example of an opening structure **149** where the tanks are each formed by an inverted hollow cup-like button structure **150** adapted to hold a hermetically thermo-sealed bladder **151** containing either the mix or wash buffer solution **152.** The tank is opened by pushing it down upon one or more prongs **153** extend upwardly from the base pan **154** of the apparatus under the footprint **155** of each of the tanks. Each prong is shaped to have an axial groove **156** which acts as an air channel to encourage the evacuation of the bladder **153** when it is punctured. Further, the length **L** of the prongs is selected to be greater than the distance **D** between the opposite top and bottom walls **157,158** of the bladder so that the prongs puncture both opposite walls causing the wash buffer to flow more rapidly out of the bladder which ensures rapid washing of the down-flow strips at the preselected appropriate time. The button has recess **154** in the form of an air conduit sized to accommodate the insertion of the tip of the prong and to thereby facilitate evacuation of the bladder. Each prong is also shaped to have an upstream oriented convex prow **159** to prevent collection of buffer solution behind the prong.

Figs 17-18 show an alternate embodiment of an opening structure where the mix tank **160** and wash tank **161** are formed by a pair of bladders secured atop the base pan **163.** Each bladder is opened by being crushed by a respective button **164,165.** Each button has a substantially flat lower surface **166** that moves toward the base pan during the opening process. The mix buffer tank **160** has a pair of discharge tubes **167** running down the base pan on either side of the wash buffer tank **161** so that the mix buffer is not trapped behind the wash buffer tank. The wash buffer tank **161** has a least one discharge tube **168** running down the center of the base pan. The use of discharge tubes allows for a more predictable dispensing of the buffer from the respective tank. Otherwise the tank may rupture in an unpredictable way sending buffer in an unintended direction to be trapped.

Figs. 19-20 show an alternate embodiment of an opening structure where the respective tank **170** is formed by an inverted, cup-like button structure **171** having a cavity **172** carrying a bladder **173** similarly to the embodiment of Figs. 15-16. However, in this embodiment the frangible bladder **173** is opened by being crushed over a pedestal **174** extending upwardly from the base pan **175** and sized and shaped to intimately engage the cavity **172** thereby more completely crushing the bladder and forcing the buffer solution out. The interrupted, diffused, down-flow test can rapidly provide an analytical panel or profile of antigen or antibody detection, and confirm the biochemical or pathogenic condition such as HIV infection, or early stage cancer prior to metastasis, or acute cardiac disorder by way of a simple, inexpensive and disposable apparatus that can be manipulated safely by a relatively low skilled person. The quality of the clinical performance of this novel platform technology surpasses previous rapid testing technologies, such as Latex particle agglutination, Flow-Through test, and the currently wide-spread Lateral Flow apparatuses. It is a technology of Rapid Confirmatory Immunological Testing (RCIT).

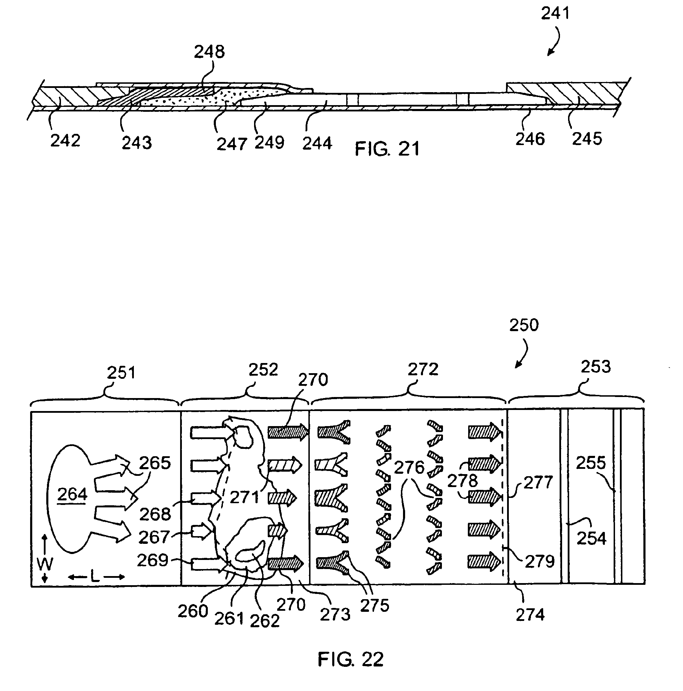

Referring now to Fig. 21, there is shown a strip (not part of the invention) wherein the flow-diffusing structure is implemented on a lateral flow chromatographic immunoassay test strip **241** having a porous sample pad **242,** adjacent to a porous conjugate pad **243** coated with a now dried reagent containing a mobilizable, labeled binding member for the analyte in question. Those pads adjacent to one another are said to be in fluid flow contact with one another. The strip has a reaction membrane **244,** and an absorption pad **245,** mounted atop a fluid impermeable vinyl backing **246.** A diffusive pad structure **247** is mounted between a portion **248** of the conjugate pad and a portion **249** of the reaction membrane thereby separating the conjugate pad and reaction membrane from one another.

Referring now to Fig. 22, there is shown a top view of a stylized lateral flow chromatographic test strip **250** having a length direction **L** and a width direction **W,** a porous sample pad **251,** adjacent to a porous conjugate pad **252,** and a reaction membrane **253** having T-line **254** and a C-line **255** zones. Again similarly, the conjugate pad **252** has been coated with a now dried reagent containing a mobilizable, labeled binding member for the analyte in question. Due to technical limitations and manufacturing inconsistencies, there exists variation in the concentration of the dried reagent as indicated by the regions **260,261,262** of the conjugate pad. Region **260** shows the weakest concentration, while region **262** shows greatest concentration. A fluid specimen **264** having chemical dis-uniformities is deposited on the sample pad **251** and begins flowing **265** laterally toward the conjugate pad **252.** Because of other manufacturing inconsistencies, the density of the particles in the pads is not uniform, leading to differences in flow rate at various locations of the conjugate pad. These differences are indicated by differently sized arrows **267,268,269.** The smallest arrow **267** shows the weakest flow, whereas the largest arrow **269** shows the strongest flow. In this example, these differential flows exist across the width and length of the conjugate pad. These conditions result in some flows **270** exhibiting a greater apparent degree of first affinity binding per unit fluid, whereas other flows **271** exhibit a lesser apparent degree of first affinity binding. The darkness of the shading of the flow arrows schematically indicates the degree of apparent first affinity binding.

The strip of Fig. 22 includes a diffusive, interrupting pad **272** located between a portion **273** of the conjugate pad **252** and a portion **274** of the reaction membrane **253** thereby separating the conjugate pad and reaction membrane from one another. In this way there is no direct fluid flow contact between the conjugate pad and the reaction membrane, but rather the fluid flow must pass through the diffusive pad before reaching the reaction membrane.

The function of the diffusive pad **272** of the flow of fluid is primarily three-fold. Firstly, the porous diffusive pad acts to cause the flow to separate at the fluid front into a plurality of branches or furcations **275** which, when these furcations converge and join together again, do so from different directions. The convergence from different directions **276** causes a mixing across the fluid front and hence the entire specimen as it flows forward. This mixing can cause the break-up of clumps of non-analyte molecules which may carry mobilizable labeled binding members, to reduce false positives. The mixing also reduces the differences in the concentrations of non-analyte molecules and labeled analyte complexes so that they are spread more evenly. Once the fluid front reaches the far end **277** of the diffusive pad, the concentrations have superior uniformity as indicated by the similar shading of the flow arrows **278.** This uniformity leads directly to giving the labeled analyte complexes a greater opportunity to form the second affinity binding and thereby reducing false negatives.

Secondly, the diffusive action of the diffusive pad **272** automatically delays or interrupts the flow from the conjugate pad **252** to the reaction membrane **253** providing more incubation time for the first affinity binding to occur, thereby increasing the overall sensitivity and specificity of the test.

Thirdly, the diffusive action of the diffusive pad causes the fluid front **279** to be more even or planar in shape so that the fluid specimen enters the reaction membrane **253** more evenly across the width of its first end **274,** substantially parallel with the second affinity binding zones **254,255** and tends to arrive at the second affinity binding zones simultaneously across the width of the device. In this way the diffuse pad provides a means for evening the fluid front passing therethrough.

It should be noted that the surfactant in the dry portion of the diffusive pad naturally provides, through capillary surface tension forces, a greater affinity for fluid to flow further into the diffusive pad rather than into the adjacent downstream structure such as the reaction membrane. In this way, the surfactant treated diffusive pad provides a means to divert the initial fluid front and cause a more even and uniformly mixed fluid front crossing into the reaction membrane, which enhances the sensitivity and specificity of the affinity bindings as described above.

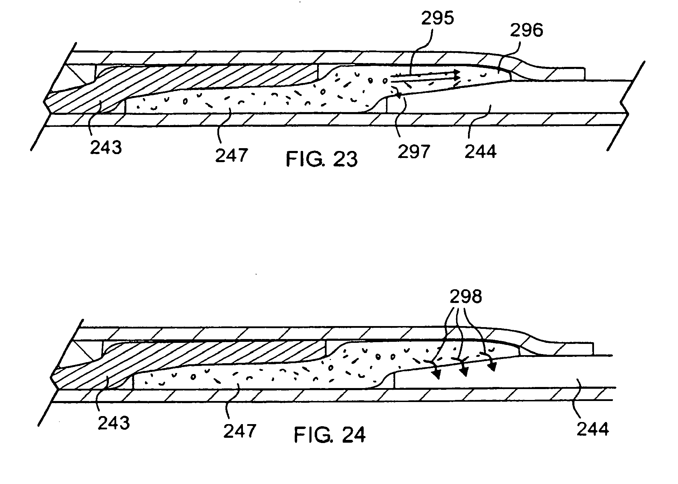

Referring now to Fig. 23, the fluid flow being driven primarily through capillary forces will tend toward fully saturating the diffusive pad before any substantial flow leaves the diffusive pad **247** for the reaction membrane **244.** This preferential fluid flow direction is primarily due to the differential surface tension caused by the surfactant treatment of the structures in the diffusive pad. Arrows **295** indicate that the predominant flow will be first toward the dry, downstream portion **296** of the diffusive pad rather than across the border **297** between the diffusive pad and the reaction membrane **244.** In this way the diffusive pad acts partially as a reservoir for delaying or interrupting the flow into the reaction membrane giving more time for first affinity binding to occur. Depending on the chemistry of the test being performed and the viscosity of the sample being tested, the flow can be delayed between about 1 and about 40 seconds. For relatively low viscosity samples such as urine, the flow is delayed for between about 2 and about 3 seconds.

Referring now to Fig. 24, as the diffusive pad **247** eventually becomes saturated and the fluid pressure, due to continued capillary action, builds at the border, the fluid front will then pass through the border into the reaction membrane **244.** This relatively rapid breakdown of the surface tension barrier at the border causes further mixing and leads to a more even fluid front. Further, because of the overlapping structure of the diffusive pad **247** to the reaction membrane **244,** the direction of flow of the fluid front makes a downward turn **298** to flow into the reaction membrane. This change in direction also serves to better mix the fluid. To cause this downward turn, the border **297** between the diffusive pad and the reaction membrane should preferably be an overlapping border. The overlap can be, for example, 25, 30, 40, 50, 60, 75, or even more than 80% of the length of the diffusive pad.

It should be noted that pretreatment with a surfactant is preferred in many cases but is not required. Pretreatment of the diffusive pad can also be in the form of pH conditioning chemicals, and non-specific adhesive blocking molecules which will selectively filter or "block-out" unwanted non-analyte adhesion molecules, typically proteins, which can non-specifically interfere and/or compete with the analyte in question in either or both of the first and second affinity binding stages of the lateral flow test. Another acceptable pretreatment condition is to have no pretreatment.

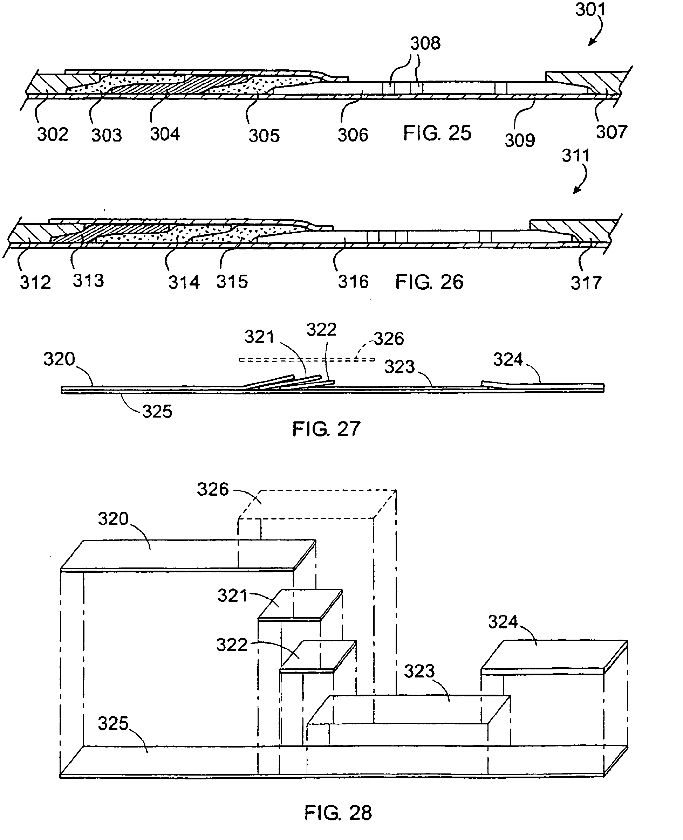

Referring now to Fig. 25 there is shown a test strip **301** adapted for use on a lateral flow rapid chromatographic immunoassay test device having a porous sample pad **302** adjacent to a first, porous upstream diffusive pad **303,** which is adjacent to a conjugate pad **304** coated with a dried reagent containing a mobilizable, labeled binding member for the analyte in question. The diffusive pad being upstream from the conjugate pad will help to premix the fluid specimen to provide for more accurate first affinity binding to occur, and potentially to break up clumping of non-analyte molecules which can lead to false mobilization of labeled binding members. The conjugate pad is also adjacent to a second, downstream diffusive pad **305** which is adjacent to the upstream end of a reaction membrane **306** which is adjacent at its downstream end to an absorption pad **307.** These pad structures are secured atop a fluid impermeable plastic backing **309.** Those pads adjacent to one another are said to be in fluid flow contact with one another. In this embodiment the two diffusive pads **303,305** straddle the conjugate pad **304.**

The first diffusive pad may be pretreated with a dried surfactant similar to one of the above embodiments. In addition to filtering out larger particles in the fluid sample, the porous first diffusive pad would then provide a more even fluid front to the conjugate pad, helping provide a more uniform first affinity binding even as the fluid front passes out of the conjugate pad.

In another example the first, upstream diffusive pad can have a pretreatment condition which includes a pH conditioning chemical, also in a dried form, which can change the pH of the fluid sample to a more optimal range for first affinity binding to occur. Similarly, the first diffusive pad **303** can also be pretreated to include non-specific adhesive blocking molecules to further filter out undesirable molecules prior to first affinity binding. Similar to the embodiment of Fig. 21, the second pad **305,** downstream from the conjugate pad **304** would have a pretreatment condition of having a dried surfactant therein.

It should be noted that the increased accuracy provides the ability to make strips having multiple "T-line" zones **308** for detecting different analytes in a single specimen such as for detecting multiple major epitopes, i.e., antigenic determinants of a single pathogen, or pathogenic organism such as bacteria, virus, parasites, rickettsia, and the like, with higher sensitivity and specificity compared to that of prior devices. It can also have multiple "T-lines" for detecting a set of bio-medical markers such as Troponin I, CK-MB, CRP and myoglobin for an endogenious disorder such as AMI with higher accuracy performance.

Referring now to Fig. 26 there is shown another alternate strip wherein a test strip **311** has a porous sample pad **312** adjacent to a conjugate pad **313** coated with a dried reagent containing a mobilizable, labeled binding member for the analyte in question. The conjugate pad is adjacent to a first, porous upstream diffusive pad **314,** which is adjacent to second, downstream diffusive pad **315** which is adjacent to the upstream end of a reaction membrane **316** which is adjacent at its downstream end to an absorption pad **317.** Those pads adjacent to one another are said to be in fluid flow contact with one another. In this embodiment the upstream diffusive pad **314** can have a different pretreatment condition than the downstream diffusive pad **315.** For example, the upstream pad can be pretreated to have a pH conditioner; and the downstream pad be pretreated to have a non-specific adhesive blocking molecule and a surfactant.

By splitting the pretreatment chemicals between two or more pads, the strip designer is not only given increased accuracy, but also more control and predictability over the reactions occurring at any given stage of the lateral flow process. Also, using two or more pretreated pads potentially avoids manufacturing inconsistencies such as the second pretreatment chemical dislodging some of the first pretreatment chemical.

It should be noted that in either of the multi-diffusive pad embodiments, no pretreatment can be a pretreatment condition. For example, the upstream pad **303** in Fig. 25 may simply be a fiberglass filter pad without any surfactant, pH conditioner or non-specific adhesive blocking molecule. In this case its pretreatment condition would be "unpretreated". This pretreatment condition would be different from the pretreatment condition of the downstream pad **305** which could have a dried surfactant pretreatment condition.

Again it should be noted that the increased accuracy provides the ability to make multiline strips for detecting different analytes in a single specimen such as for detecting multiple major epitopes, i.e., antigenic determinants of an endogenious disorder such as AMI, or a single exogenous pathogen, or pathogenic organism such as bacteria, viruses such as HIV, parasites, rickettsia, and the like.

Referring now to Figs. 27 and 28, the strip can be formed using a simplified manufacturing process which merely overlaps the individual porous material elements or pads. For example, a series of separated elements such as a sample pad **320,** a conjugate pad **321,** a diffusive interrupting pad **322,** a reaction membrane **323,** and an absorption pad **324,** are attached to an elongated backing strip of a semi-rigid, fluid impermeable material **325,** such as vinyl and the like. A plastic tab (or tape) **326** is optionally used to protect the conjugate pad, and optionally to cover a portion of the diffusive pad, the reaction membrane and the sample pad. The sample pad, conjugate pad, diffusive pad, reaction membrane, and absorption pad are in direct lateral fluid flow contact with each other such that the direction of fluid flow in the test device is from the sample pad laterally (i.e., by capillary force or wicking action) toward the end of the absorption pad. As shown in Fig. 28, each pad may be laminarly and overlappingly placed upon the backing and secured in place to form the strip.

Depending on the disease being tested and the condition of the fluid specimen, many of the above embodiments have been found to achieve an accuracy of at least 99.9%.

While the exemplary embodiment have been described, modifications can be made and other embodiments may be devised without departing from the scope of the appended claims.

## Claims

1. A flow immunoassay testing apparatus for testing a fluid specimen for the presence of an analyte, wherein said apparatus (1) includes a source (conjugate pad) of labelled conjugate adapted to carry a binding member molecule bindable to said analyte, and a reaction membrane carrying a capture binding member molecule bindable to said analyte, wherein said apparatus comprises:
a first chamber shaped and dimensioned to accept said specimen and be subjectable to a supply of mix buffer solution and said source of labelled conjugate to form a fluid mixture;
said first chamber leading to a flow interrupting incubation reservoir with a dam for accumulating said fluid mixture;
a second chamber holding at least one test result signal generator responsive to an amount of said analyte bonded to said labelled conjugate, said generator comprising a chromatographic test strip including said reaction membrane oriented in an oblique, downward flow orientation, said membrane having at least one test zone;
said dam comprising a porous, diffusive structure located on a fluid flow path between said conjugate source and said reaction membrane, for creating a plurality of convergent fluid paths between said conjugate source and said membrane.

2. The apparatus of claim 1 wherein said strip is formed without a source of mobilizable labelled binding members.

3. The apparatus of claim 1 or claim 2 wherein said generator comprises a plurality of test zones adapted to provide a measurable basis for a quantitative result display.

4. The apparatus according to any preceding claim which further comprises means for triggering a dispensing of said supply into said first chamber.

5. The apparatus according to any preceding claim which further comprises an openable tank shaped and dimensioned to releasably hold said supply, wherein said tank in an open condition is in fluid communication with said dam.

6. The apparatus of any preceding claim wherein said diffusive structure has a first pre-treatment condition.

7. The apparatus according to Claim 6 wherein said diffusive structure is pretreated with a pre-treatment surfactant.

8. The apparatus according to claim 6 wherein said diffusive structure is pre treated with a pH conditioner.

9. The apparatus of any preceding claim wherein said diffusive structure is made from material having a plurality of intersecting surface structures, wherein a junction between first and second of said structures is not substantially parallel.

10. The apparatus of any preceding claim wherein said diffusive structure comprises a material selected from the group consisting of: fibreglass, cellulose and fibrous plastic and the like.

11. The apparatus of any of Claims 6 to 8 comprising a second diffusive structure, with a pre-treatment condition different to that of the diffusive structure.

12. The apparatus of any preceding claim comprising fluid flow through said reaction membrane under the combined forces of gravity, siphoning and capiliarity.

13. The apparatus of any preceding claim wherein said supply of mix buffer solution has a volume between about 200 microlitres and about 300 microlitres.

14. The apparatus of any preceding claim which further comprises a second supply of wash buffer solution having a volume between about 2.5 milliliters and about 3 milliliters.

15. The apparatus of any preceding claim wherein said apparatus is a lateral flow chromatographic immunoassay strip, wherein said conjugate source is a conjugate pad and said first diffusive structure being a first diffusive pad located between a portion of said conjugate pad and said portion of said reaction membrane.

16. The apparatus of Claim 15, wherein said portion of said conjugate pad overlaps said first diffusive pad, and a portion of said first diffusive pad overlaps said portion of said reaction membrane.

17. A method of operating a flow immunoassay test for testing a fluid specimen for the presence of an analyte with apparatus including a source (conjugate pad) of labelled conjugate adapted to carry a binding member molecule bindable to said analyte, and a reaction membrane carrying a capture binding member molecule bindable to said analyte, a first chamber shaped and dimensioned to accept said specimen and be subjectable to a supply of mix buffer solution and said source of labelled conjugate to form a fluid mixture, said first chamber leading to a flow interrupting incubation reservoir with a dam for accumulating said fluid mixture, said dam comprising a porous, diffusive structure located on a fluid flow path between said conjugate source and said reaction membrane, a second chamber holding at least one test result signal generator responsive to an amount of said analyte bonded to said labelled conjugate, said generator comprising a chromatographic test strip including said reaction membrane oriented in an oblique, downward flow orientation, said membrane having at least one test zone;
the method comprising creating a plurality of convergent fluid paths between said conjugate source and said membrane, for promoting first affinity binding prior to the second chamber and
interrupting down-flow of fluid on a fluid flow path between a portion of said conjugate source and a portion of said reaction membrane.

18. The method of Claim 17 comprising providing a diffusive structure comprising a pH conditioner.

19. The method of Claim 18 comprising providing a second diffusive structure with a pre-treatment condition different to that of the diffusive structure.

20. The method of any of Claims 17 to 19 comprising adjusting or regulating a buffer solution flow to limit overflow of fluid to the second chamber.

21. The method of any of Claims 17 to 20 comprising diverting an initial fluid front passing through the diffusive structure(s).

## Patentansprüche

1. Durchfluss-Immunoanalyseprüfvorrichtung zum Prüfen einer Flüssigkeitsprobe auf Vorhandensein eines Analyten, wobei die Vorrichtung eine Quelle (Konjugatblock) von markiertem Konjugat, die zum Enthalten eines Bindeelementmoleküls ausgelegt ist, das sich an den Analyten binden kann, und eine Reaktionsmembran umfasst, welche ein Abfang-Bindeelementmolekül trägt, das sich an den Analyten binden kann, wobei die Vorrichtung Folgendes umfasst:
eine erste Kammer, die so geformt und dimensioniert ist, dass sie die Probe aufnehmen kann und einer Zufuhrmenge der Mischpufferlösung und der Quelle des markierten Konjugats ausgesetzt werden kann, um eine Flüssigkeitsmischung zu bilden;
wobei die erste Kammer zu einem den Durchfluss unterbrechenden Inkubationsbehälter mit einem Damm zum Ansammeln der Flüssigkeitsmischung führt;
eine zweite Kammer, die mindestens einen Prüfergebnissignalgenerator enthält, der auf eine Menge des Analyten anspricht, welche an das markierte Konjugat gebunden ist, wobei der Generator einen chromatographischen Teststreifen umfasst, der die Reaktionsmembran einschließt, die schräg, stromabwärts ausgerichtet ist, wobei die Membran mindestens einen Prüfbereich hat;
wobei der Damm eine poröse Diffusionsstruktur, die sich auf einem Fluidweg zwischen der Konjugatquelle und der Reaktionsmembran befindet, zum Erzeugen mehrerer konvergenter Fluidwege zwischen der Konjugatquelle und der Membran umfasst.

2. Vorrichtung nach Anspruch 1, wobei der Streifen ohne Quelle von mobilisierbaren markierten Bindungselementen gebildet ist.

3. Vorrichtung nach Anspruch 1 oder Anspruch 2, wobei der Generator mehrere Prüfbereiche umfasst, welche zum Bereitstellen einer messbaren Basis für eine quantitative Ergebnisanzeige ausgelegt sind.

4. Vorrichtung nach einem der vorherigen Ansprüche, die ferner Mittel zum Auslösen der Zufuhr zur ersten Kammer umfasst.

5. Vorrichtung nach einem der vorherigen Ansprüche, die ferner einen zu öffnenden Tank umfasst, welcher so geformt und dimensioniert ist, dass er freigebbar die Zufuhrmenge aufnehmen kann, wobei der Tank im offenen Zustand sich in Fluidverbindung mit dem Damm befindet.

6. Vorrichtung nach einem der vorherigen Ansprüche, wobei die Diffusionsstruktur einen ersten Vorbehandlungszustand besitzt.

7. Vorrichtung nach Anspruch 6, wobei die Diffusionsstruktur mit einem Vorbehandlungstensid vorbehandelt ist.

8. Vorrichtung nach Anspruch 6, wobei die Diffusionsstruktur mit einem pH-Zusatzstoff vorbehandelt ist.

9. Vorrichtung nach einem der vorherigen Ansprüche, wobei die Diffusionsstruktur aus einem Material hergestellt ist, welches mehrere sich schneidende Oberflächenstrukturen hat, wobei eine Berührungsfläche zwischen der ersten und zweiten Struktur im Wesentlichen nicht parallel ist.

10. Vorrichtung nach einem der vorherigen Ansprüche, wobei die Diffusionsstruktur ein Material umfasst, das aus der Gruppe bestehend aus folgenden ausgewählt wird: Glasfasern, Zellulose und faserhaltige Kunststoffe und dergleichen.

11. Vorrichtung nach einem der Ansprüche 6 bis 8, welche eine zweite Diffusionsstruktur mit einem Vorbehandlungszustand umfasst, der sich von dem der Diffusionsstruktur unterscheidet.

12. Vorrichtung nach einem der vorherigen Ansprüche, die eine Flüssigkeitsströmung durch die Reaktionsmembran unter den vereinten Kräften der Schwerkraft, der Absaugkraft und der Kapillarität umfasst.

13. Vorrichtung nach einem der vorherigen Ansprüche, wobei die Zufuhr der Mischpufferlösung ein Volumen zwischen etwa 200 Mikrolitern und etwa 300 Mikrolitern hat.

14. Vorrichtung nach einem der vorherigen Ansprüche, die ferner eine zweite Zufuhr von Waschpufferlösung umfasst, welche ein Volumen zwischen etwa 2,5 Millilitern und etwa 3 Millilitern hat.

15. Vorrichtung nach einem der vorherigen Ansprüche, wobei die Vorrichtung ein chromatographischer Immunoteststreifen mit seitlichem Ablauf ist, wobei die Konjugatquelle ein Konjugatblock ist und die erste Diffusionsstruktur ein erster Diffusionsblock ist, welcher sich zwischen einem Abschnitt des Konjugatblocks und dem Abschnitt der Reaktionsmembran befindet.

16. Vorrichtung nach Anspruch 15, wobei der Abschnitt des Diffusionsblocks sich mit dem ersten Diffusionsblock überlappt und ein Abschnitt des ersten Diffusionsblocks mit dem Abschnitt der ersten Reaktionsmembran überlappt.

17. Verfahren zum Betreiben einer Durchfluss-Immunoanalyse zum Prüfen einer Flüssigkeitsprobe auf Vorhandensein eines Analyten mit Vorrichtung, das eine Quelle (Konjugatblock) von markiertem Konjugat, welche zum Enthalten eines Bindeelementmoleküls ausgelegt ist, das sich an einen Analyten binden kann, und eine Reaktionsmembran umfasst, die ein Abfang-Bindeelementmolekül trägt, das sich an den Analyten binden kann, eine erste Kammer, die so geformt und dimensioniert ist, dass sie die Probe aufnehmen kann und einer Zufuhr einer Mischpufferlösung und der Quelle des markierten Konjugats ausgesetzt werden kann, um eine Flüssigkeitsmischung zu bilden, wobei die erste Kammer zu einem die Strömung unterbrechenden Inkubationsbehälter mit einem Damm zum Ansammeln der Flüssigkeitsmischung führt, wobei der Damm eine poröse Diffusionsstruktur umfasst, welche sich auf einem Fluidweg zwischen der Konjugatquelle und der Reaktionsmembran befindet, eine zweite Kammer, die mindestens einen Prüfergebnissignalgenerator aufweist, welcher auf eine Zufuhrmenge des Analyten reagiert, die an das markierte Konjugat gebunden ist, wobei der Generator einen chromatographischen Teststreifen einschließlich der Reaktionsmembran umfasst, die schräg, stromabwärts ausgerichtet ist, wobei die Membran mindestens einen Prüfbereich hat;
wobei das Verfahren zur Förderung der ersten Affinitätsbindung vor der zweiten Kammer und zum Unterbrechen des Abflusses von Flüssigkeit auf einem Fluidweg zwischen einem Abschnitt der Konjugatquelle und einem Abschnitt der Reaktionsmembran mehrere konvergente Fluidwege zwischen der Konjugatquelle und der Membran erzeugt.

18. Verfahren nach Anspruch 17, das das Bereitstellen einer Diffusionsstruktur umfasst, die ein pH-Zusatzmittel umfasst.

19. Verfahren nach Anspruch 18, welches das Bereitstellen einer zweiten Diffusionsstruktur mit einem Vorbehandlungszustand umfasst, der sich von dem der Diffusionsstruktur unterscheidet.

20. Verfahren nach einem der Ansprüche 17 bis 19, das das Einstellen oder Regulieren einer Pufferlösungsströmung umfasst, um das Überlaufen von Flüssigkeit in die zweite Kammer zu begrenzen.

21. Verfahren nach einem der Ansprüche 17 bis 20, das ein Verteilen einer anfänglichen Flüssigkeitsfront umfasst, die durch die Diffusionsstruktur(en) läuft.

## Revendications

1. Appareil de test d'immunoessai à écoulement pour tester un spécimen fluide quant à la présence d'un analyte, dans lequel ledit appareil inclut une source (tampon de conjugué) de conjugué marqué adaptée pour porter une molécule à élément de liaison, liable audit analyte, et une membrane de réaction portant une molécule à élément de liaison par capture, liable audit analyte, dans lequel ledit appareil comprend :
une première enceinte façonnée et dimensionnée pour accepter ledit spécimen et être susceptible d'être soumise à une alimentation en solution tampon mixte et à ladite source de conjugué marqué pour former un mélange fluide ;
ladite première enceinte conduisant à un réservoir d'incubation interrompant le flux avec un barrage pour accumuler ledit mélange fluide ;
une seconde enceinte contenant au moins un générateur de signal de résultat d'essai réagissant à une quantité dudit analyte lié audit conjugué marqué, ledit générateur comprenant une bande d'essai chromatographique incluant ladite membrane de réaction orientée dans une orientation oblique en flux descendant, ladite membrane ayant au moins une zone d'essai ;
ledit barrage comprenant une structure poreuse diffusive se trouvant sur un chemin d'écoulement fluide entre ladite source de conjugué et ladite membrane de réaction, pour créer une pluralité de chemins fluides convergents entre ladite source de conjugué et ladite membrane.

2. Appareil selon la revendication 1, dans lequel ladite bande est formée sans source d'éléments de liaison marqués mobilisables.

3. Appareil selon la revendication 1 ou la revendication 2, dans lequel ledit générateur comprend une pluralité de zones d'essai adaptées pour fournir une base mesurable pour un affichage de résultat quantitatif.

4. Appareil selon l'une quelconque des revendications précédentes, qui comprend en outre des moyens pour déclencher une distribution de ladite alimentation dans ladite première enceinte.

5. Appareil selon l'une quelconque des revendications précédentes, qui comprend en outre un réservoir ouvrable, façonné et dimensionné pour contenir ladite alimentation de façon libérable, dans lequel ledit réservoir dans un état ouvert est en communication fluide avec ledit barrage.

6. Appareil selon l'une quelconque des revendications précédentes, dans lequel ladite structure diffusive a un premier état de prétraitement.

7. Appareil selon la revendication 6, dans lequel ladite structure diffusive est prétraitée avec un agent tensioactif de prétraitement.

8. Appareil selon la revendication 6, dans lequel ladite structure diffusive est prétraitée avec un conditionneur de pH.

9. Appareil selon l'une quelconque des revendications précédentes, dans lequel ladite structure diffusive est faite d'un matériau ayant une pluralité de structures de surface se croisant, dans lequel une jonction entre la première et la seconde desdites structures n'est pas essentiellement parallèle.

10. Appareil selon l'une quelconque des revendications précédentes, dans lequel ladite structure diffusive comprend un matériau choisi dans le groupe constitué par : une fibre de verre, la cellulose et une matière plastique fibreuse et similaires.

11. Appareil selon l'une quelconque des revendications 6 à 8, comprenant une seconde structure diffusive, avec un état de prétraitement différent de celui de la structure diffusive.

12. Appareil selon l'une quelconque des revendications précédentes, comprenant un écoulement fluide à travers ladite membrane de réaction sous les forces combinées de gravité, de siphonnement et de capillarité.

13. Appareil selon l'une quelconque des revendications précédentes, dans lequel ladite alimentation en solution tampon mixte a un volume entre environ 200 microlitres et environ 300 microlitres.

14. Appareil selon l'une quelconque des revendications précédentes, qui comprend en outre une seconde alimentation en solution tampon de lavage ayant un volume entre environ 2,5 millilitres et environ 3 millilitres.

15. Appareil selon l'une quelconque des revendications précédentes, dans lequel ledit appareil est une bande d'immunoessai chromatographique à flux latéral, dans lequel ladite source de conjugué est un tampon de conjugué et ladite première structure diffusive qui est un premier tampon diffuseur se trouve entre une partie dudit tampon de conjugué et ladite partie de ladite membrane de réaction.

16. Appareil selon la revendication 15, dans lequel ladite partie dudit tampon de conjugué chevauche ledit premier tampon diffuseur, et une partie dudit premier tampon diffuseur chevauche ladite partie de ladite membrane de réaction.

17. Procédé de mise en oeuvre d'un test d'immunoessai à écoulement pour tester un spécimen fluide quant à la présence d'un analyte avec un appareil incluant une source (tampon de conjugué) de conjugué marqué adaptée pour porter une molécule à élément de liaison, liable audit analyte, et une membrane de réaction portant une molécule à élément de liaison par capture, liable audit analyte, une première enceinte façonnée et dimensionnée pour accepter ledit spécimen et être susceptible d'être soumise à une alimentation en solution tampon mixte et à ladite source de conjugué marqué pour former un mélange fluide, ladite première enceinte conduisant à un réservoir d'incubation interrompant le flux avec un barrage pour accumuler ledit mélange fluide, ledit barrage comprenant une structure poreuse diffusive se trouvant sur un chemin d'écoulement fluide entre ladite source de conjugué et ladite membrane de réaction, une seconde enceinte contenant au moins un générateur de signal de résultat d'essai réagissant à une quantité dudit analyte lié audit conjugué marqué, ledit générateur comprenant une bande d'essai chromatographique incluant ladite membrane de réaction orientée dans une orientation oblique en flux descendant, ladite membrane ayant au moins une zone d'essai ;
le procédé comprenant la création d'une pluralité de chemins fluides convergents entre ladite source de conjugué et ladite membrane, pour favoriser la liaison par première affinité avant la seconde enceinte et interrompre l'écoulement descendant de fluide sur un chemin d'écoulement fluide entre une partie de ladite source de conjugué et une partie de ladite membrane de réaction.

18. Procédé selon la revendication 17, comprenant la fourniture d'une structure diffusive comprenant un conditionneur de pH.

19. Procédé selon la revendication 18, comprenant la fourniture d'une seconde structure diffusive avec un état de prétraitement différent de celui de la structure diffusive.

20. Procédé selon l'une quelconque des revendications 17 à 19, comprenant l'ajustement ou la régulation d'un flux de solution tampon pour limiter le débordement de fluide à la seconde enceinte.

21. Procédé selon l'une quelconque des revendications 17 à 20, comprenant la diversion d'un front de fluide initial passant à travers la (les) structure(s) diffusive(s).
